(19) Europäisches Patentamt
European Patent Office
Office européen des brevets

(11) **EP 2 389 147 B1**

(12) **EUROPÄISCHE PATENTSCHRIFT**

(45) Veröffentlichungstag und Bekanntmachung des Hinweises auf die Patenterteilung:
**12.04.2017 Patentblatt 2017/15**

(21) Anmeldenummer: **10702268.3**

(22) Anmeldetag: **21.01.2010**

(51) Int Cl.:
*A61F 9/008* *(2006.01)*          *A61F 9/009* *(2006.01)*

(86) Internationale Anmeldenummer:
**PCT/EP2010/050701**

(87) Internationale Veröffentlichungsnummer:
**WO 2010/084163 (29.07.2010 Gazette 2010/30)**

(54) **VORRICHTUNG UND VERFAHREN ZUM ERZEUGEN VON STEUERDATEN ZUR OPERATIVEN FEHLSICHTIGKEITSKORREKTUR EINES AUGES**

DEVICE AND METHOD FOR PRODUCING CONTROL DATA FOR THE SURGICAL CORRECTION OF DEFECTIVE EYE VISION

DISPOSITIF ET PROCÉDÉ DE PRODUCTION DE DONNÉES DE COMMANDE POUR LA CORRECTION OPÉRATOIRE D'UN DÉFAUT DE VISION D'UN OEIL

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **21.01.2009   DE 102009005482**

(43) Veröffentlichungstag der Anmeldung:
**30.11.2011   Patentblatt 2011/48**

(73) Patentinhaber: **Carl Zeiss Meditec AG**
**07745 Jena (DE)**

(72) Erfinder:
• **STOBRAWA, Gregor**
**07743 Jena (DE)**
• **BISCHOFF, Mark**
**07749 Jena (DE)**
• **BERGT, Michael**
**99425 Weimar (DE)**

(74) Vertreter: **Patentanwälte Geyer, Fehners & Partner mbB**
**Perhamerstrasse 31**
**80687 München (DE)**

(56) Entgegenhaltungen:
WO-A1-2005/011546     WO-A1-2005/011547
WO-A2-2009/124668     DE-A1-102005 013 558
DE-A1-102006 053 120     US-A- 5 891 131
US-A1- 2004 169 820     US-A1- 2008 319 428

**Beschreibung**

Die Erfindung bezieht sich auf ein Verfahren zur Erzeugung von Steuerdaten, die zur Ansteuerung einer Lasereinrichtung zur operativen Fehlsichtigkeitskorrektur eines Auges eines Patienten ausgebildet sind, wobei im Betrieb der Lasereinrichtung die Hornhaut durch Anpressen ihrer Vorderfläche an eine Kontaktfläche verformt ist, wobei im Verfahren eine auf die unverformte Hornhaut bezogene Korrekturfläche vorgegeben ist, die zur Fehlsichtigkeitskorrektur als Schnittfläche in der Hornhaut zu erzeugen ist, und wobei im Verfahren die im Betrieb der Lasereinrichtung vorliegende Verformung der Hornhaut durch eine Transformation der Koordinaten für Punkte in der unverformten Hornhaut in Koordinaten derselben Punkte in der verformten Hornhaut berücksichtigt wird.

Die Erfindung bezieht sich weiter auf eine Vorrichtung zur Erzeugung von Steuerdaten, die zur Ansteuerung einer Lasereinrichtung zur operativen Fehlsichtigkeitskorrektur eines Auges eines Patienten ausgebildet sind, wobei im Betrieb der Lasereinrichtung die Hornhaut durch Anpressen ihrer Vorderfläche an eine Kontaktfläche verformt ist und wobei eine auf die unverformte Hornhaut bezogene Korrekturfläche vorgegeben ist, die zur Fehlsichtigkeitskorrektur als Schnittfläche in der Hornhaut zu erzeugen ist, und wobei die Vorrichtung bei der Erzeugung der Steuerdaten die im Betrieb der Lasereinrichtung vorliegende Verformung der Hornhaut durch eine Transformation der Koordinaten für Punkte in der unverformten Hornhaut in Koordinaten derselben Punkte in der verformten Hornhaut berücksichtigt.

Der klassische Weg zur Korrektur der Fehlsichtigkeit des menschlichen Auges ist die Brille. Mittlerweile wird jedoch auch vermehrt refraktive Chirurgie eingesetzt, die durch Veränderung der Augenhornhaut eine Fehlsichtigkeitskorrektur bewirkt. Ziel der Operationsmethoden ist es dabei, die Hornhaut gezielt zu verändern, um so die Lichtbrechung zu beeinflussen. Hierfür sind unterschiedliche Operationsmethoden bekannt. Am verbreitetsten ist gegenwärtig die sogenannte Laser-Insitu-Keratomileusis, die auch LASIK abgekürzt wird. Dabei wird zuerst eine Hornhautlamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp. Irvine, USA, vertrieben wird. Nachdem die Lamelle gelöst und zur Seite geklappt wurde, ist bei der LASIK-Operation die Anwendung eines Excimer-Lasers vorgesehen, der das derart freigelegte Hornhautgewebe durch Ablation abträgt. Nachdem auf diese Art und Weise in der Hornhaut liegendes Volumen verdampft wurde, wird die Hornhautlamelle wieder auf den ursprünglichen Platz zurückgeklappt.

Die Anwendung eines Laserkeratoms zum Freilegen der Lamelle ist vorteilhaft, da die Infektionsgefahr dadurch verringert und die Schnittqualität vergrößert ist. Insbesondere kann die Lamelle mit sehr viel konstanterer Dicke hergestellt werden. Auch ist der Schnitt potentiell glatter, was spätere optische Störungen durch diese auch nach der Operation verbleibende Grenzfläche mindert.

Zur Schnitterzeugung wird eine Serie optischer Durchbrüche an vorbestimmten Stellen so erzeugt, daß dadurch die Schnittfläche ausgebildet wird. Beim Laserkeratom bildet die Schnittfläche die vor dem Einsatz der Laserablation abzuklappende Lamelle.

Bei der herkömmlichen LASIK-Methode wird freigelegtes Hornhautgewebe verdampft, was auch als "Schleifen" der Hornhaut mittels Laserstrahlung bezeichnet wird. Die Volumenentfernung, die für eine Fehlsichtigkeitskorrektur notwendig ist, wird dabei für jedes Flächenelement der freigelegten Hornhaut durch die Zahl der Laserpulse und deren Energie eingestellt. Man sieht deshalb in der LASIK-Methode für den Ablationslaser ein sogenanntes shot file vor, das für verschiedene Punkte auf der Augenhornhaut festlegt, wie oft der Laserstrahl auf definierte Punkte auf der Hornhaut gerichtet werden soll und mit welcher Energie. Die Volumenentfernung wurde dabei heuristisch ermittelt, nicht zuletzt da sie sehr von der Ablationswirkung des Laserstrahls, mithin von der Wellenlänge, Fluence etc. der eingesetzten Strahlung abhängt. Auch spielt der Zustand der Augenhornhaut eine Rolle; hier ist insbesondere der Feuchtigkeitsgehalt der Augenhornhaut zu nennen. Die WO 96/11655 schildert eine Vorrichtung und ein Verfahren für die LASIK-Methode. Dabei wird insbesondere eine Formel angegeben, die aus dem vor-operativen Hornhautkrümmungsradius und der gewünschten Dioptrienkorrektur den zu erreichenden Hornhautkrümmungsradius berechnet. Eine ähnliche Berechnung ist in der EP 1153584 A1 beschrieben - ebenfalls für die Hornhautablation mittels LASIK.

Die US 5993438 schlägt vor, ein Volumen aus der Hornhaut durch Verdampfen und Absorption in der Hornhaut zu entfernen.

Die WO 2005/092172 offenbart, wie Brechkraftmessungen, die in einer Ebene ermittelt wurden, in eine andere Ebene transformiert werden können. Die Schrift erwähnt, daß dieses Vorgehen für verschiedene Augenbehandlungen verwendet werden kann, insbesondere für die lasergestützte Ablation.

Ein weiteres laserbasiertes, augenchirurgisches Verfahren liegt darin, das zu entfernende Hornhautvolumen nicht zu verdampfen, sondern durch einen Laserschnitt zu isolieren. Das Volumen wird also nicht mehr abladiert, sondern in der Hornhaut durch eine dreidimensionale Schnittfläche isoliert und somit entnehmbar gemacht. Für solche Verfahren sind Erfahrungswerte, die zum Schleifen der Hornhaut mittels Ablationslaserstrahlung entwickelt wurden, nicht brauchbar. Statt dessen werden Steuerdaten für den Betrieb des Lasers zur Isolation des zu entfernenden Hornhautvolumens benötigt. In der US 6110166 und der US 7131968 ist ein solches augenchirurgisches Verfahren beschrieben. Dabei sind in US 6110166 verschiedene Volumenformen gezeigt, und es ist erwähnt, daß der Fachmann das passende Volumen auswählen kann.

Die DE 102006053118 A1 schildert die Erzeugung von Steuerdaten für die volumenisolierende Fehlsichtigkeitskorrektur.

Aus der DE 102006053120 A1 und der DE 102006053119 A1 der Carl Zeiss Meditec AG ist es bekannt, bei der Erzeugung solcher Steuerdaten von Fehlsichtigkeitsdaten auszugehen, welche die Brechkraft einer für die Fehlsichtigkeitskorrektur tauglichen Brille angeben. Auch ist es aus dieser Druckschrift, die somit ein gattungsgemäßes Verfahren und eine gattungsgemäße Vorrichtung schildert, bekannt, Daten zu verwenden, die auch eine Astigmatismuskorrektur oder Korrekturen höherer Aberrationsordnungen bewirken. Der aus der DE 102006053120 A1 bekannte Ansatz erreicht durch die Verwendung von Fehlsichtigkeitsdaten, die für eine herkömmliche Brillenkorrektur gedacht sind, eine erhebliche Vereinfachung bei der präoperativen Augenvermessung, da die Erzeugung von Brillenkorrekturdaten in der Ophthalmologie tägliche Praxis ist. Diese Vereinfachung bedingt allerdings auch eine gewisse Beschränkung der möglichen Korrekturergebnisse, weil zwangsläufig nur Korrekturen erreicht werden können, die auch mit einer normalen Brille möglich wären. Hierbei ist auch zu berücksichtigen, daß Korrekturen, wie sie z.B. mit Gleitsichtbrillen möglich sind, für den Ansatz gemäß DE 102006053120 A1 ausscheiden, da solche Korrekturen immer davon ausgehen, daß die Sehachse je nach Blickrichtung an unterschiedlichen Stellen durch das Brillenglas fällt, was es möglich macht, für unterschiedliche Blickrichtungen (z.B. beim mehr nach unten gerichteten Lesen oder dem mehr in die Entfernung gerichteten Weitsehen) unterschiedliche optische Eigenschaften der Brille zur Wirkung bringen zu können. Dies ist bei der refraktiven Chirurgie an der Hornhaut nicht anwendbar, da durch die Augenbewegung die Augenhornhaut sich bei der Änderung der Blickrichtung selbstverständlich mitbewegt. Es gibt also, anders als bei einem Brillenglas, keine Veränderung des Durchtrittspunktes der optischen Achse durch die Hornhaut, wenn der Augapfel rotiert. Der aus der DE 102006053120 A1 bekannte Ansatz kann also folglich nur vergleichsweise einfache Brillen-Fehlsichtigkeitskorrekturdaten als Eingangsgröße bei der Steuerdaten verwenden - mit der Konsequenz entsprechend begrenzter Korrekturmöglichkeiten.

Für die volumenisolierende Fehlsichtigkeitskorrektur ist die Präzision, mit der die erforderlichen Schnittflächen erzeugt werden, von großer Bedeutung. Anders als beim Laserkeratom hat die Lage der Schnittflächen direkte Auswirkung auf die Qualität der optischen Korrektur. Bei der herkömmlichen LASIK-Methode ist dagegen ausschließlich die Präzision, mit der die Laserablation betrieben wird, für die Güte der optischen Korrektur wichtig. Dies erkennt man schon daraus, daß die Erzeugung der Hornhautlamelle in einer großen Vielzahl von Operationen mit einem vergleichsweise grob arbeitenden, mechanischen Messer praktiziert wird bzw. wurde.

Da für die Präzision der Schnittflächenerzeugung die exakte Positionierung des Auges wichtig ist, schlägt der Stand der Technik, beispielsweise die WO 2005/011547 A1 vor, daß bei laserchirurgischen Vorrichtungen ein Kontaktglas verwendet werden kann, an das die Hornhaut angepreßt wird. Dieses Kontaktglas dient dazu, das Auge zu fixieren.

Für die Präzision der Schnittflächen ist aber nicht nur die präzise Lage des Auges wichtig, auch die Form der Hornhaut muß bekannt sein. Da diese von Patient zu Patient innerhalb gewisser Bereiche variiert, dient das Kontaktglas auch dazu, der Hornhautvorderfläche eine feste, definierte Form zu geben. Beim Anpressen der Hornhautvorderseite an das Kontaktglas kommt es folglich zu einer Deformation der Hornhaut, die je nach Abweichung der Kontaktglaskrümmung von der natürlichen Hornhautkrümmung des jeweiligen Patienten unterschiedlich groß ist.

Ist die Lage der Schnittflächen für die optische Korrektur von Bedeutung, d. h. wird nicht nur eine Lamelle isoliert und das zu entfernende Volumen durch Ablation entfernt, ist die Deformation der Hornhaut bei der Bestimmung der Zielkoordinaten für die Schnittflächenerzeugung wesentlich. Es ist aus der US 2008/0319428 A1 und deren Familienmitglied WO 2008/055706 A bekannt, die Deformation dadurch zu berücksichtigen, daß die vorher ermittelten Zielpunkte einer Koordinatentransformation unterzogen werden. In der genannten WO-Veröffentlichung wird diese Transformation als "Anpreßtransformation" bezeichnet und es werden Transformationsgleichungen für eine Kombination aus sphärischem Kontaktglas und sphärischer Hornhautvorderfläche gegeben. Die DE 102008017293 A1 der Carl Zeiss Meditec AG ergänzt diese Transformationsgleichungen, so daß auch bei andersartigen Kontaktglas- und Hornhautkrümmungen die Koordinatentransformation vorgenommen werden kann.

Die Erfindung betrifft also das Konzept, eine Korrektur der optischen Abbildungsfehler des menschlichen Auges dadurch durchzuführen, daß mittels Laserstrahlung in der Augenhornhaut eine Separierung eines Gewebevolumens erreicht wird, welches dann aus der Hornhaut entfernt wird. Dadurch wird eine gezielte Änderung der Brechkraft der Augenhornhaut erreicht. Diese Änderung erfolgt lokal, d. h. in dem Bereich der Hornhaut, aus dem das Gewebevolumen entnommen wird. Üblicherweise orientiert man sich dabei an der Pupille des Auges.

Die Entnahme des separierten Volumens verändert die Geometrie, nämlich die Krümmung der Hornhautoberfläche. Damit eine gewünschte Fehlsichtigkeitskorrektur erreicht wird, muß deshalb das separierte und zu entnehmende Volumen hinsichtlich seiner Form spezielle Eigenschaften aufweisen.

In Anlehnung an das klassische LASIK-Verfahren, wird üblicherweise das separierte Volumen durch drei Grenzflächen umschrieben. Eine anteriore Grenzfläche wird in konstantem Abstand unter der Augenhornhaut ausgebildet. Dies ist dann besonders einfach, wenn die Hornhaut mit einem flachen Kontaktglas applaniert wird. Da diese Schnittfläche in die Richtung am vordersten liegt, wird sie als anteriore Fläche oder in Anlehnung an das bekannte LASIK-Verfahren als Flapfläche bezeichnet.

Weiter ist das Volumen durch eine tiefer liegende Schnittfläche begrenzt, die als posteriore Schnittfläche oder, da das Volumen als Lentikel aufgefaßt werden kann, als Lentikelfläche bezeichnet wird. Es wird dafür gesorgt, daß das insgesamt zu entnehmende Volumen die Krümmung der Hornhautvorderfläche ändert. Eine der beiden Flächen, meist die posteriore, hat i. d. R. eine Geometrie, die ausschlaggebend für die Fehlsichtigkeitskorrektur ist.

Prinzipiell könnte man daran denken, die anteriore und die posteriore Fläche so zu gestalten, daß sie eine gemeinsame Schnittlinie haben. Dies ist zum einen bei einer WeitsichtigkeitsKorrektur nicht möglich, da dort das zu entnehmende Volumen in der Mitte, d. h. im Bereich der Sehachse, dünner sein muß, als am Rand. Zum anderen möchte man aus operativen Gründen auch bei einer Kurzsichtigkeits-Korrektur eine gewisse Mindestdicke des Volumens am Rande sicherstellen, um es einfach zu entnehmen können. Die anteriore Fläche und die posteriore Fläche werden deshalb über eine sogenannte Lentikelrandfläche verbunden.

Durch diese drei Schnittflächen ist das separierte Volumen entnehmbar gemacht, da dann das Volumen vollständig oder nahezu vollständig durch die Schnittflächen umschlossen ist. Die absolute Lage und relative Ausdehnung der Flächen in der Hornhaut legen die Zone fest, innerhalb der die optische Wirkung nach Entnahme des separierten Volumens zwischen diesen Flächen eintritt. Hier orientiert man sich, wie bereits erwähnt, an der Pupille. Dieser Ansatz führt dazu, daß die beiden Schnittflächen, nämlich die anteriore und die posteriore Schnittfläche, von denen eine oder beide optisch wirksam sein können, zu einem geschlossenen Volumen verbunden werden müssen, das eine geeignete Lage innerhalb der Hornhaut haben muß.

Der Erfindung liegt die Aufgabe zugrunde, für die laserchirurgische Fehlsichtigkeitskorrektur, bei der für das Korrekturergebnis wesentliche Korrekturflächen als Schnittfläche im Auge ausgebildet werden, ein Verfahren sowie eine Vorrichtung zur Berechnung von Steuerdaten anzugeben, mit denen beliebige Korrekturflächen verwendet werden können und dennoch den Einflüssen eines Kontaktglases ausreichend Rechnung getragen wird.

Diese Aufgabe wird gemäß der Erfindung gelöst durch ein Verfahren zur Erzeugung von Steuerdaten, die zur Ansteuerung einer Lasereinrichtung zur operativen Fehlsichtigkeitskorrektur eines Auges eines Patienten ausgebildet sind, wobei im Betrieb der Lasereinrichtung die Hornhaut durch Anpressen ihrer Vorderfläche an eine Kontaktfläche verformt ist, wobei im Verfahren eine auf die unverformte Hornhaut bezogene Korrekturfläche vorgegeben ist, die zur Fehlsichtigkeitskorrektur als Schnittfläche in der Hornhaut zu erzeugen ist, und wobei im Verfahren die im Betrieb der Lasereinrichtung vorliegende Verformung der Hornhaut durch eine Transformation der Koordinaten für Punkte in der unverformten Hornhaut in Koordinaten derselben Punkte in der verformten Hornhaut berücksichtigt wird, wobei

a) mehrere in der Korrekturfläche oder in einer daraus abgeleiteten Näherungsfläche liegende Punkte ausgewählt werden und deren Koordinaten mittels der Transformation transformiert werden, um transformierte Punkte zu erhalten,
b) durch Interpolation an die transformierten Punkte eine Interpolationsfläche angepaßt wird und
c) auf der Interpolationsfläche (I') liegende Zielpunkte ausgewählt und für die Erzeugung der Steuerdaten verwendet werden.

Die Aufgabe wird weiter gemäß der Erfindung gelöst durch eine Vorrichtung zur Erzeugung von Steuerdaten, die zur Ansteuerung einer Lasereinrichtung zur operativen Fehlsichtigkeitskorrektur eines Auges eines Patienten ausgebildet sind, wobei im Betrieb der Lasereinrichtung die Hornhaut durch Anpressen ihrer Vorderfläche an eine Kontaktfläche verformt ist und wobei eine auf die unverformte Hornhaut bezogene Korrekturfläche vorgegeben ist, die zur Fehlsichtigkeitskorrektur als Schnittfläche in der Hornhaut zu erzeugen ist, und wobei die Vorrichtung bei der Erzeugung der Steuerdaten die im Betrieb der Lasereinrichtung vorliegende Verformung der Hornhaut durch eine Transformation der Koordinaten für Punkte in der unverformten Hornhaut in Koordinaten derselben Punkte in der verformten Hornhaut berücksichtigt, wobei

a) die Vorrichtung bei der Erzeugung der Steuerdaten mehrere in der Korrekturfläche oder in einer daraus abgeleiteten Näherungsfläche liegende Punkte auswählt und deren Koordinaten mittels der Transformation transformiert, um transformierte Punkte zu erhalten,
b) die Vorrichtung bei der Erzeugung der Steuerdaten durch Interpolation an die transformierten Punkte eine Interpolationsfläche anpaßt und
c) die Vorrichtung bei der Erzeugung der Steuerdaten auf der Interpolationsfläche liegende Zielpunkte auswählt und für die Erzeugung der Steuerdaten verwendet.

Die Erfindung geht von der Situation aus, daß die Hornhaut deformiert wird, wenn der Hornhautvorderfläche eine bestimmte Form gegeben wird, in dem das eingangs erwähnte Kontaktglas angepreßt wird. Weiter geht die Erfindung davon aus, daß die Deformation der Hornhaut durch eine Koordinatentransformation wiedergegeben werden kann, die die Koordinaten von Punkten in der unverformten Hornhaut in transformierte Koordinaten derselben Punkte in der deformierten Hornhaut umsetzt.

Aufbauend auf diese bekannten Transformationen sieht die Erfindung nun vor, daß beliebig geformte Korrekturflächen, die als Schnittflächen in der Augenhornhaut erzeugt werden sollen, geeignet behandelt werden, um die Wirkung der Hornhautdeformation zu berücksichtigen. Dabei ermöglicht das Vorgehen gemäß der zweiten Variante der Erfindung, beliebige Korrekturflächen zu behandeln. Insbesondere ist keine Beschränkung auf sphärische Flächen mehr gegeben, wie sie bislang im Stand der Technik angesprochen waren. Dadurch können auch für beliebige, insbesondere nicht-rotationssymmetrische Schnittflächen, die Hornhautdeformation berücksichtigt werden. Während sphärische Korrekturflächen oder Korrekturflächen, die eine Kombination aus einer Sphäre und einem Zylinderanteil darstellen (wie sie in der Astigmatismuskorrektur bekannt sind), hinsichtlich der Koordinatentransformation analytisch behandelt werden können und man im wesentlichen nur den Scheitel und einen Randpunkt für eine Sphäre bzw. zwei Randpunkte für eine Kombination aus Sphäre und Zylinder für die Transformation berücksichtigen muß, ist bei Korrekturflächen, die höhere Ordnungen korrigieren oder auch nicht-rotationssymmetrisch sind, ein solcher Ansatz nicht möglich. Auch solche Korrekturflächen können mit dem erfindungsgemäßen Prinzip hinsichtlich der Hornhautdeformation behandelt werden.

Dieses Vorgehen erlaubt es, beliebig geformte Korrekturflächen zu verwenden und dennoch für diese Korrekturflächen die Auswirkung der Verformung der Hornhaut zu berücksichtigen. Es ist auch nicht mehr zwingend erforderlich, die Krümmung des Kontaktglases möglichst präzise an die Krümmung der natürlichen Hornhaut anzupassen, um verformungsbedingte Fehler gering zu halten, wenn von den üblichen sphärischen oder sphärisch-zylindrischen Formen bei der Korrekturfläche abgewichen werden soll.

Weiter erlaubt die durch Interpolation (oder ggf. Approximation) erhaltene Interpolationsfläche I' die Festlegung von Bahnkurven, die eine schnelle Bearbeitung zulassen. Würde man hingegen die Zielpunkte selbst oder eine Bahnkurve, welche die Zielpunkte verbindet, transformieren, wäre nicht automatisch sichergestellt, daß die transformierten Zielpunkte bzw. die transformierte Bahnkurve automatisch immer noch hinsichtlich der Bearbeitungsgeschwindigkeit optimal wäre. Aufgrund der nahezu beliebigen Verzerrungen, die durch die Anpreßtransformation verursacht werden können, wäre vielmehr davon auszugehen, daß das Optimum nicht mehr erreicht ist.

Je nach Definition der Korrekturfläche kann eine recht große Anzahl an charakteristischen Punkten nötig werden, um die Interpolation nach der Transformation der charakteristischen Punkte mit ausreichender Genauigkeit durchführen zu können. Für solche Fälle kann eine Weiterbildung der Erfindung vorteilhaft sein, die zuerst eine Näherungsfläche aus der Korrekturfläche ableitet. Dabei kann ein Glättungsvorgang oder auch ein Approximationsvorgang eingesetzt werden. Die Glättung kann insbesondere eine Tiefpaßfilterung sein. Auch ist eine Approximation durch eine analytisch einfacher beschreibbare Näherungsfläche möglich. Einen besonderen Vorteil erhält man, wenn man eine Näherungsfläche wählt, die eine 2-, 3- oder 4-zählige Winkelsymmetrie aufweist. Der Transformation wird dann nicht die eigentliche Korrekturfläche, sondern die Näherungsfläche unterworfen. Um die Unterschiede zwischen der Näherungsfläche und der Korrekturfläche zu berücksichtigen wird weiter eine Abweichung zwischen der Korrekturfläche und der Näherungsfläche ermittelt. Diese Abweichung wird zweckmäßigerweise an den charakteristischen Punkten bestimmt, die für die Transformation ausgewählt wurden. Es ist aber auch möglich, eine allgemeine Abweichungsfunktion oder eine Abweichungsmatrix zu ermitteln. Mit dieser Abweichung, die der Transformation nicht unterworfen wird, wird dann die Interpolationsfläche korrigiert. Dieser Ansatz geht also davon aus, daß die Abweichung hinsichtlich der Transformation im wesentlichen invariant ist. Der Rechenaufwand kann in dieser Ausbildungsform erheblich reduziert werden.

Die für die Transformation auszuwählenden Punkte berücksichtigen vorzugsweise Symmetrien der Korrekturfläche. Weiter ist es zweckmäßig, Invarianzen der Anpreßtransformation bei der Wahl der zu transformierenden Punkte, die auch als charakteristische Punkte angesehen werden können, zu berücksichtigen.

Einer Weiterbildung der Erfindung liegt zudem die Aufgabe zugrunde, eine gattungsgemäße Vorrichtung $_{bzw.}$ ein gattungsgemäßes Verfahren dahingehend weiterzubilden, daß Steuerdaten für die operative Fehlsichtigkeitskorrektur möglichst rechensparsam erzeugt werden können und zugleich auch komplexere Korrekturen realisierbar sind.

Die Aufgabe wird in der Weiterbildung mit einer Vorrichtung zur Erzeugung von Steuerdaten zur Ansteuerung einer Lasereinrichtung zur operativen Fehlsichtigkeitskorrektur eines Auges eines Patienten, wobei die Steuerdaten zur Ansteuerung einer Lasereinrichtung ausgebildet sind, welche durch Einstrahlen von Laserstrahlung in die Hornhaut des Auges Hornhaut-Gewebe trennt, die Vorrichtung die Steuerdaten so erstellt, daß die Lasereinrichtung bei Betrieb gemäß den Steuerdaten die Laserstrahlung so abgibt, daß ein Volumen in der Hornhaut isoliert ist, dessen Entfernung aus der Hornhaut die gewünschte Fehlsichtigkeitskorrektur bewirkt, und die Vorrichtung zur Ermittlung der Steuerdaten einen Krümmungsradius berechnet, den die um das Volumen verminderte Hornhaut hat gelöst, bei der der Krümmungsradius $R_{CV}^*$ ortsabhängig ist und folgender Gleichung genügt:

$$R_{CV}^*(r,\varphi) = 1 / ( (1/R_{CV}(r,\varphi)) + B_{COR}(r,\varphi)/ (n_c\text{-}1) ) + F,$$

wobei $R_{CV}(r,\varphi)$ der lokale Krümmungsradius der Hornhaut vor Entfernung des Volumens , $n_c$ die Brechzahl des Materials der Hornhaut, F ein Faktor ist, und $B_{COR}(r,\varphi)$ die für die gewünschte Fehlsichtigkeitskorrektur nötige, lokale Brechkraftänderung in einer im Scheitelpunkt der Hornhaut liegenden Ebene ist, wobei es mindestens zwei Radien r1 und r2 gibt, für die $B_{COR}(r=r1,\varphi) \neq B_{COR}(r=r2,\varphi)$ gilt.

Diese Aufgabe wird in der Weiterbildung mit einem Verfahren zur Erzeugung von Steuerdaten zur Ansteuerung einer Lasereinrichtung zur operativen Fehlsichtigkeitskorrektur eines Auges eines Patienten, wobei die Steuerdaten zur Ansteuerung einer Lasereinrichtung ausgebildet sind, welche durch Einstrahlen von Laserstrahlung in die Hornhaut des Auges Hornhaut-Gewebe trennt, die Steuerdaten so erstellt werden, daß die Lasereinrichtung bei Betrieb gemäß den Steuerdaten die Laserstrahlung so abgibt, daß ein Volumen in der Hornhaut isoliert ist, dessen Entfernung aus der Hornhaut die gewünschte Fehlsichtigkeitskorrektur bewirkt, und zur Ermittlung der Steuerdaten ein Krümmungsradius berechnet wird, den die um das Volumen verminderte Hornhaut hat, gelöst, wobei der Krümmungsradius $R_{CV}^*$ ortsabhängig ist und folgender Gleichung genügt:

$$R_{CV}^*(r,\varphi) = 1 / ( (1/R_{CV}(r,\varphi)) + B_{COR}(r,\varphi)/ (n_c\text{-}1) ) + F,$$

wobei $R_{CV}(r,\varphi)$ der lokale Krümmungsradius der Hornhaut vor Entfernung des Volumens, $n_c$ die Brechzahl des Materials der Hornhaut, F ein Faktor ist, und $B_{COR}(r,\varphi)$ die für die gewünschte Fehlsichtigkeitskorrektur nötige, lokale Brechkraftänderung in einer im Scheitelpunkt der Hornhaut liegenden Ebene ist, wobei es mindestens zwei Radien r1 und r2 gibt, für die $B_{COR}(r=r1,\varphi) \neq B_{COR}(r=r2,\varphi)$ gilt.

Die Erfindung stellt in der Weiterbildung also eine Steuergröße bzw. eine Bemessungsgröße bereit, auf deren Basis das zu entfernende Volumen und damit die dieses Volumen in der Hornhaut isolierende Schnittfläche möglichst exakt berechnet werden kann. Sie definiert eine Gleichung für den Krümmungsradius, den die Hornhaut nach der Entnahme des durch die Behandlungsvorrichtung bzw. das Verfahren isolierten Volumens haben soll. Mit dieser Gleichung ist das zu entnehmende Volumen analytisch exakt berechenbar.

Die in der Weiterbildung für die Berechnung des zu entnehmenden Volumens verwendete Gleichung unterscheidet sich bei genauer Betrachtung erheblich von dem Ansatz, wie er in der DE 102006053120 A1 verwendet wurde. Es wird nun eine andere Funktion verwendet, die nicht mehr die Brechkraft einer Brille berücksichtigt, die in Abstand zum Auge liegt, sondern eine Brechkraftverteilung, die in Kreiskoordinaten geschrieben mindestens radial variiert. Zudem liegt diese Brechkraftverteilung, mit der für das zu entnehmende Volumen der neue Krümmungsradius, den die Hornhaut nach der operativen Korrektur haben soll, berechnet wird, nicht mehr in einem Abstand zur Hornhaut, sondern gibt den Korrekturbedarf in einer Ebene an, die im Hornhautscheitel liegt. Die Erfindung greift den analytischen Ansatz der DE 102006053120 A1 auf und nimmt zugleich von den dort verwendeten Brillenkorrekturwerten Abkehr, indem nun eine radial variierende Brechkraftverteilung, welche den Korrekturbedarf in der im Hornhautscheitel liegenden Ebene wiedergibt, eingeführt wird.

Damit ist, ohne daß der Rechenaufwand signifikant gesteigert würde, eine viel weitgehendere Korrektur der Fehlsichtigkeit möglich. Beispielsweise kann nun in einem zentralen Bereich um die optische Achse, z.B. im Radius der phototopischen Pupille, ein Korrekturwert angewendet werden, der dem bisherigen Brillenkorrekturwert entspricht, und für größere Durchmesser können andere Brechkraftwerte eingesetzt werden. Damit läßt sich eine Presbyopie des Auges derart beheben, daß im zentralen Bereich, z.B. im Radius der photopischen Pupille eine Korrektur für die Nahsicht (vergleichbar einer Lesebrille) und für größere Durchmesser eine Korrektur für die Fernsicht (vergleichbar einer Fernbrille) bewirkt wird.

Das Volumen ist über die Gleichung nun so bestimmt bzw. bestimmbar, daß die Hornhaut nach Entfernung des Volumens den definierten Krümmungsradius hat. Eine besonders einfach zu berechnende und vor allem auch einfach zu realisierende (aber beileibe natürlich nicht die einzige) Definition des Volumens begrenzt das Volumen durch eine Grenzfläche, die eine anteriore und eine posteriore Teilfläche umfaßt, wobei die anteriore Teilfläche in konstantem Abstand $d_F$ zur Hornhautvorderfläche liegt. Die Begriffe "anterior" und "posterior" entsprechen der üblichen medizinischen Nomenklatur. Eine zusätzliche Randfläche kann erforderlich (bei Weitsichtigkeitskorrektur) bzw. vorteilhaft ist, um die beiden Teilflächen zu verbinden und zugleich eine Mindestranddicke zu gewährleisten.

Durch die in konstantem Abstand zur Hornhautoberfläche liegende anteriore Teilfläche ist die Ausbildung dieser Teilfläche besonders einfach. Die posteriore Teilfläche hat natürlich zwangsläufig dann keinen konstanten Abstand zur Hornhautvorderfläche. Die optische Korrektur erfolgt durch die Formgebung der posterioren Teilfläche. Durch diesen Ansatz ist der Rechenaufwand erheblich vereinfacht, da eine sphärische Teilfläche (die anteriore Teilfläche) besonders einfach zu berechnen ist und der Rechenaufwand auf die Bestimmung der posterioren Teilfläche konzentriert ist. Bei einem derartigen Ansatz hat die posteriore Teilfläche einen Krümmungsverlauf, der bis auf eine additive Konstante identisch mit dem Krümmungsverlauf der Hornhautvorderfläche nach Entnahme des Volumens sein kann. In die Konstante fließt der Abstand, den die anteriore Teilfläche von der Hornhautvorderfläche hält, ein.

Die in der Weiterbildung vorhandene radiale Abhängigkeit der Brechkraftverteilung bedeutet, daß, in Polarkoordinaten gesehen, es für alle Winkel mindestens zwei Radien gibt, bei denen unterschiedliche Werte der Brechkraftverteilung vorliegen.

Die verwendete Brechkraftverteilung kann als Ergebnis einer Berechnung unter Verwendung von Wellenfrontvermessung oder Topographiemessung der Hornhautvorderseite der Augenhornhaut vorliegen. Demgemäß sieht die erfindungsgemäße Gleichung, von welcher die Berechnung des Hornhautvolumens ausgeht, auch einen lokalen Krümmungsradius der Hornhaut vor. Das dabei gewählte Koordinatensystem ist vorzugsweise auf den Hornhautscheitel bezogen.

Ist die Topographie $Z_{CV}:_R{}^2 \rightarrow R^3$ (Menge aller Punkte $Z_{CV}(r,\varphi)$ die auf der Hornhautvorderseite liegen) bekannt, so läßt sich der lokale Krümmungsradius $R_{CV}(z(r,\varphi))$ beispielsweise durch eine beste Anpassung einer Kugelfläche mit Radius R an die Fläche $Z_{CV}$ in einem infinitesimalen Radius um den Punkt $z_{CV}(r,\varphi)$ bestimmen. Auch kann nur die Anpassung eines Krümmungskreises in radialer Richtung verwendet werden. Dann gilt:

$$R_{CV}(r,\varphi) = \frac{\sqrt{1+\left(\dfrac{\partial}{\partial r}z_{CV}(r,\varphi)\right)^2}}{\left|\dfrac{\partial^2}{\partial r^2}z_{CV}(r,\varphi)\right|}$$

Auf diese Weise erhält man mittels der Gleichung die gewünschte Verteilung des Krümmungsradius der Hornhautvorderseite $R_{CV}^*(r, \varphi)$, welche durch die refraktive Korrektur $B_{COR}(r, \varphi)$ erreicht werden soll.

Die ortsabhängige refraktive Korrektur $B_{COR}(r, \varphi)$ kann auch in der Form einer Wellenfront repräsentiert sein. Diese Wellenfronten werden in der Praxis oft durch Zernike-Polynome beschrieben. D.h. die mathematische Beschreibung der Refraktionskorrektur durch lokal veränderliche Brechkräfte lassen sich direkt in einen Satz von Zernike-Koeffizienten umrechnen. Dem Fachmann sind dazu geeignete Methoden bekannt. Als Beispiel sei hier auf die Bestimmung der Brillenäquivalente der Sphäre/Zylinder/Achse aus den Zernike-Koeffizienten $Z_{2,i}$ und sphärische Abberation (repräsentiert durch $Z_{4,0}$) verwiesen. Diese Umrechung läßt sich analog auf weitere Zernike-Ordungen ausdehnen, die dann zu einer allgemeinen lokal veränderlichen Brechkraft führt, wie sie in der Fachwelt auch als Fehler höherer Ordnung bekannt sind (z.B. Koma).

Das Dickenprofil $\Delta z(r,\varphi)$ des zu entnehmenden Volumens ist nun so bestimmt bzw. bestimmbar, daß die Topographie $z_{CV}^*(r,\varphi)$ der Hornhaut nach der Entfernung des Volumens den lokalen Krümmungsradius $R_{CV}^*(r, \varphi)$ aufweist; dabei gilt:

$$z_{CV}^*(r, \varphi) = z_{CV}(r, \varphi) - \Delta z(r, \varphi).$$

Wird ein isoliertes Volumen aus der Hornhaut entfernt, so ist $\Delta z(r,\varphi)$ stets positiv. Dies ist aber keine notwendige Bedingung für die Korrektur. Es ist ebenfalls möglich, die refraktive Korrektur und damit verbunden die Veränderung der Krümmung der Hornhautvorderseite durch das Einbringen eines zusätzlichen Volumens in die Hornhaut zu verändern. In diesem Fall ist dann $\Delta z(r,\varphi)$ immer negativ. Auch gemischte Fälle sind möglich, bei denen $\Delta z(r,\varphi)$ sowohl positive als auch negative Anteile hat. Praktisch ist dies der Fall, wenn beispielsweise eine geringe refraktive Korrektur für die Fernsicht bei Myopie durch Extraktion von Gewebe und gleichzeitig eine Korrektur der Presbyopie durch Implantation einer kleinen Linse im zentralen Bereich der optischen Zone erfolgen soll. In diesem Fall kann die Dicke des Implantats durchaus größer als die Dicke des für die Myopiekorrektur zu entfernenden Gewebevolumens sein und damit $\Delta z(r,\varphi)$ im Zentralbereich positive und im Randbereich negative Werte haben.

Das Dickenprofil $\Delta z(r,\varphi)$ des Volumens ergibt sich aus der Differenz der Topographien. Ist die gewünschte Topographie nach der Korrektur $z_{CV}^*(r,\varphi)$ bekannt, so ist auch das Dickenprofil bestimmt.

Der Fachmann kann nun analytisch oder mittels geeigneter numerischer Methoden $z_{CV}^*(r,\varphi)$ aus $R_{CV}^*(r,\varphi)$ durch zweimalige Integration über die Fläche bestimmen. Die dabei anfallenden beiden Integrationskonstanten werden so gewählt, daß beispielsweise der gewünschte Behandlungsdurchmesser für die refraktive Korrektur entsteht und gleichzeitig das zu entnehmende Volumen minimiert wird.

Insbesondere ist es in der Weiterbildung bevorzugt, daß die Vorrichtung bei der Ermittlung der Steuerdaten die lokale Brechkraftänderung $B_{COR}(r,\varphi)$ so festlegt, daß ein charakteristischer Radius $r_{ch}$ besteht, für den die Radialfunktion der Brechkraftänderung $B_{COR}(r,\varphi)$ stückweise konstant ist, für den also $B_{COR}(r<r_{ch},\varphi=const) = B_a \neq B_b = B_{COR}(r>r_{ch},\varphi=const)$ gilt.

Die zur Korrektur verwendete Brechkraftverteilung kann, wie bereits erwähnt, in bestimmten Pupillenbereichen, z.B. einem Zentralbereich sowie einem Randbereich, unterschiedliche Werte aufweisen, um eine optische Korrektur zu erreichen, die auch bei stark variierenden Sehbedingungen optimale Ergebnisse erzielt bzw. individuell, z.B. im Fall der Altersweitsichtigkeit (Presbyopie), optimal angepaßt ist.

Insbesondere ist es bevorzugt, daß die Vorrichtung bei der Ermittlung der Steuerdaten die lokale Brechkraftänderung $B_{COR}(r,\varphi)$ so festlegt, daß ein charakteristischer Radius $r_{ch}$ besteht, für den die Radialfunktion der Brechkraftänderung $B_{COR}(r,\varphi)$ stückweise konstant ist, für den also $B_{COR}(r<r_{ch},\varphi=const) = B_a \neq B_b = B_{COR}(r>r_{ch},\varphi=const)$ gilt.

Zwischen den stückweise konstanten Werten der Brechkraftänderungen kann ein kontinuierlicher Übergang erfolgen. Für diese Ausgestaltung ist es deshalb zweckmäßig, daß die Vorrichtung bei der Ermittlung der Steuerdaten die lokale Brechkraftänderung $B_{COR}(r,\varphi)$ so festlegt, daß zwei Radien $r_a$ und $r_b$ bestehen, für die die Radialfunktion der Brechkraftänderung $B_{COR}(r,\varphi)$ stückweise konstant ist, für die also $B_{COR}(r<r_a,\varphi=const) = B_a \neq B_b = B_{COR}(r>r_b,\varphi=const)$ gilt, wobei die Radialfunktion der Brechkraftänderung $B_{COR}(r,\varphi)$ im Übergangsbereich zwischen $r_a$ und $r_b$ kontinuierlich von $B_a$ nach $B_b$ übergeht.

Die lokale Brechkraftänderung $B_{COR}(r,\varphi)$ kann als Spezialfall Symmetrien aufweisen, die es erlauben, die Koordinatenabhängigkeiten zu separieren. Das ermöglicht beispielsweise folgende Darstellungen bei der Steuerwerterzeugung:

$$B_{COR}(r,\varphi) = B_1(r) \cdot B_2(\varphi) \text{ (multiplikativer Separationsansatz)}$$

$$B_{COR}(r,\varphi) = B_1(r) + B_2(\varphi) \text{ (additiver Separationsansatz)}.$$

Ein Spezialfall der Separation ergibt sich, wenn die Brechkraftverteilung keine Winkelabhängigkeit aufweist. Da dies rechnerisch besonders einfach ist, ist es bevorzugt, daß bei der Ermittlung der Steuerdaten die lokale Brechkraftänderung winkelunabhängig festgelegt ist bzw. wird.

Hierbei ist ganz grundlegend darauf hinzuweisen, daß Brechkraft und Krümmungsradius mit einer einfachen Gleichung ineinander überführt werden können. Es gilt: $B = (n_C-1)/R$, wobei B die Brechkraft und R der für diese Brechkraft zugeordnete Radius ist. Es ist also im Rahmen der Erfindung jederzeit möglich, zwischen Radius-Betrachtungsweise und Brechkraft-Betrachtungsweise bzw. -Darstellung zu wechseln. Die bei der Ermittlung der Steuerdaten bei Brechkraft-Darstellungen zu verwendende Gleichung lautet:

$$B^*_{CV}(r,\varphi) = \cfrac{1}{\cfrac{1}{B_{CV}(r,\varphi) + B_{COR}(r,\varphi)} + \cfrac{F}{(n_C-1)}}$$

Soweit hier von dem Radius der Hornhautvorderfläche gesprochen wird, kann völlig analog auch die Brechkraft verwendet werden, so daß alle hier im Zusammenhang mit dem Radius der Hornhautvorderfläche gegebene Erläuterungen selbstverständlich analog auch für die Brechkraft-Darstellung bzw. -Sichtweise gelten, wenn R durch B gemäß dem genannten Zusammenhang ersetzt wird.

Selbstverständlich können die hier beschriebenen Merkmale beliebig miteinander kombiniert werden, solange sie sich nicht technisch widersprechen.

Die erfindungsgemäßen Verfahren aller Varianten zum Erzeugen der Steuerdaten können ohne Rückgriff auf menschliche Mitwirkung ausgeführt werden. Insbesondere können sie von einem Computer durchgeführt werden, der unter Steuerung eines erfindungsgemäßen Programms das erfindungsgemäße Verfahren ausführt und aus entsprechenden Vorgaben die Steuerdaten für die Lasereinrichtung ermittelt. Insbesondere ist bei der Ermittlung der Steuerdaten die Mitwirkung eines Arztes in keiner Weise erforderlich, da mit der Ermittlung der Steuerdaten noch kein therapeutischer Eingriff verbunden ist. Dieser findet erst bei der Anwendung der zuvor ermittelten Steuerdaten statt.

Soweit in dieser Beschreibung ein Verfahren bzw. einzelne Schritte eines Verfahrens zur Ermittlung von Steuerdaten zur optischen Fehlsichtigkeitskorrektur beschrieben wird, kann das Verfahren bzw. können einzelne Schritte des Verfahrens durch eine entsprechend ausgestaltete Vorrichtung ausgeführt werden. Analoges gilt für die Erläuterung der Betriebsweise einer Vorrichtung, die Verfahrensschritte ausführt. Insoweit sind Vorrichtungs- und Verfahrensmerkmale dieser Beschreibung äquivalent. Insbesondere ist es möglich, das Verfahren mit einem Computer zu realisieren, auf dem ein entsprechendes erfindungsgemäßes Programm ausgeführt wird. Die vorliegende Erfindung wird in den unabhängigen Ansprüchen 1 und 8 definiert. Weitere vorteilhafte Ausführungsformen gehen aus den abhängigen Ansprüchen hervor.

Die Erfindung wird nachfolgend unter Bezugnahme auf die Zeichnungen beispielshalber noch näher erläutert.

In den Zeichnungen zeigt:

Fig. 1      eine Schemadarstellung einer Behandlungsvorrichtung bzw. eines Behandlungsgerätes zur Fehlsichtigkeitskorrektur,

Fig. 2      eine Schemadarstellung hinsichtlich des Aufbaus des Behandlungsgerätes der Fig. 1,

Fig. 3      eine Prinzipdarstellung zur Einbringung gepulster Laserstrahlung in das Auge bei der Fehlsichtigkeitskorrektur mit dem Behandlungsgerät der Fig. 1,

Fig. 4      eine weitere Schemadarstellung des Behandlungsgerätes der Fig. 1,

Fig. 5      eine schematische Schnittdarstellung durch die Augenhornhaut mit Darstellung eines zur Fehlsichtigkeitskorrektur zu entfernenden Volumens,

Fig. 6      ein Schnitt durch die Augenhornhaut nach Entfernung des Volumens der Fig. 5,

Fig. 7      eine Schnittdarstellung ähnlich der Fig. 5,

Fig. 8      eine schematische Schnittdarstellung durch die Augenhornhaut zur Veranschaulichung der Volumenentnahme,

Fig. 9      ein Diagramm mit möglichen Verläufen einer Brechkraftverteilung, welche bei der Ermittlung des zu entfernenden Volumens verwendet wird,

Fig. 10     ein Ablaufdiagramm der Ermittlung des zu entfernenden Volumens,

Fig. 11     eine grafische Veranschaulichung für das geometrische Transformations-Verfahren für einen bestimmten Winkel um die z-Achse angegeben und

Fig. 12     eine grafische Veranschaulichung eines gegenüber Fig. 11 vereinfachten Transformations-Verfahrens.

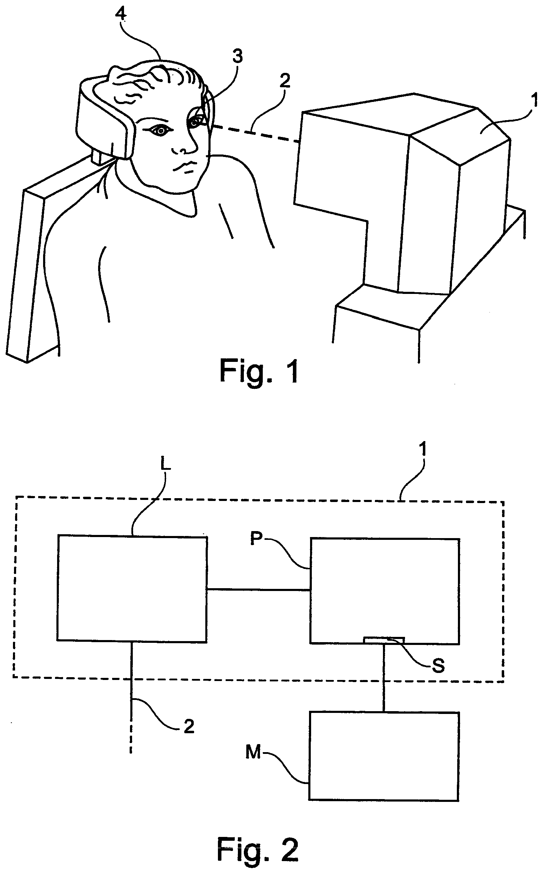

Figur 1 zeigt ein Behandlungsgerät 1 für ein augenchirurgisches Verfahren, das dem in der EP 1159986 A1 bzw. der US 5549632 beschriebenen ähnelt. Das Behandlungsgerät 1 bewirkt mittels ein Behandlungs-Laserstrahlung 2 eine Fehlsichtigkeitskorrektur an einem Auge 3 eines Patienten 4. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Abberationen höherer Ordnung umfassen. Die Behandlungs-Laserstrahlung 2 wird in der beschriebenen Ausführungsform als gepulster in das Auge 3 fokussierter Laserstrahl aufgebracht. Die Pulsdauer liegt dabei z.B. im Femtosekundenbereich, und die Laserstrahlung 2 wirkt mittels nicht-linearer optischer Effekte in der Hornhaut. Der Laserstrahl weist z.B. 50 bis 800 fs kurze Laserpulse (bevorzugt 100 - 400 fs) mit einer Pulswiederholfrequenz zwischen 10 und 500 kHz auf. Die Baugruppen des Gerätes 1 werden im beschriebenen Ausführungsbeispiel von einer integrierten Steuereinheit gesteuert, die aber natürlich auch eigenständig ausgebildet sein kann.

Vor dem Einsatz des Behandlungsgerätes wird die Fehlsichtigkeit des Auges 3 mit einer oder mehreren Meßeinrichtungen vermessen.

Figur 2 zeigt schematisch das Behandlungsgerät 1. Es weist in dieser Variante mindestens zwei Einrichtungen oder Module auf. Eine Lasereinrichtung L gibt den Laserstrahl 2 auf das Auge 3 ab. Der Betrieb der Lasereinrichtung L erfolgt dabei vollautomatisch, d.h. die Lasereinrichtung L startet auf ein entsprechendes Startsignal hin die Ablenkung des Laserstrahls 2 und erzeugt dabei Schnittflächen, die auf noch zu beschreibende Art und Weise aufgebaut sind und ein Volumen in der Augenhornhaut isolieren. Die den Betrieb erforderlichen Steuerdaten empfängt die Lasereinrichtung L zuvor von einer Planungseinrichtung P als Steuerdatensatz über nicht näher bezeichnete Steuerleitungen. Die Übertragung findet vor dem Betrieb der Lasereinrichtung L statt. Natürlich kann Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

Vorzugsweise wird der Steuerdatensatz zum Behandlungsgerät 1 übertragen und weiter vorzugsweise ist ein Betrieb der Lasereinrichtung L gesperrt, bis an der Lasereinrichtung L ein gültiger Steuerdatensatz vorliegt. Ein gültiger Steuerdatensatz kann ein Steuerdatensatz sein, der prinzipiell zur Verwendung mit der Lasereinrichtung L der Behandlungsvorrichtung 1 geeignet ist. Zusätzlich kann die Gültigkeit aber auch daran geknüpft werden, daß weitere Prüfungen bestanden werden, beispielsweise ob im Steuerdatensatz zusätzlich niedergelegte Angaben über das Behandlungsgerät 1, z. B. eine Geräteseriennummer, oder den Patienten, z. B. eine Patientenidentifikationsnummer, mit anderen Angaben übereinstimmen, die beispielsweise an der Behandlungsvorrichtung ausgelesen oder separat eingegeben wurden, sobald der Patient in der korrekten Stellung für den Betrieb der Lasereinrichtung L ist.

Die Planungseinheit P erzeugt den Steuerdatensatz, der der Lasereinheit L zur Ausführung der Operation zur Verfügung gestellt wird, aus Meßdaten und Fehlsichtigkeitsdaten, die für das zu behandelnde Auge ermittelt wurden. Sie werden der Planungseinheit P über eine Schnittstelle S zugeführt und stammen im dargestellten Ausführungsbeispiel aus einer Meßeinrichtung M, die das Auge des Patienten 4 zuvor vermessen hat. Natürlich kann die Meßeinrichtung M auf beliebige Art und Weise die entsprechenden Meß- und Fehlsichtigkeitsdaten an die Planungseinrichtung P übermitteln.

Die Übertragung kann mittels Speicherchips (z.B. per USB oder memory stick), Magnetspeichern (z.B. Disketten), per Funk (z.B. WLAN, UMTS, Bluetooth) oder drahtgebunden (Z.B. USB, Firewire, RS232, CAN-Bus, Ethernet etc.) erfolgen. Gleiches gilt natürlich hinsichtlich der Datenübertragung zwischen Planungseinrichtung P und Lasereinrichtung L.

Eine direkte Funk- oder Draht-Verbindung der Meßeinrichtung M mit der Behandlungseinrichtung 1 hinsichtlich der Datenübertragung, die in einer Variante verwendet werden kann, hat den Vorteil, daß die Verwendung falscher Meß- und Fehlsichtigkeitsdaten mit größtmöglicher Sicherheit ausgeschlossen ist. Dies gilt insbesondere dann, wenn die Überführung des Patienten von der Meßeinrichtung M bzw. den Meßeinrichtungen zur Lasereinrichtung L mittels einer (in der Figur nicht dargestellten) Lagerungseinrichtung erfolgt, die mit der Meßeinrichtung M bzw. der Lasereinrichtung L so zusammenwirkt, daß die jeweiligen Einrichtungen erkennen, ob der Patient 4 in der jeweiligen Position zum Vermessen bzw. Einbringen der Laserstrahlung 2 ist. Mit einem Verbringen des Patienten 4 von der Meßeinrichtung M zur Lasereinrichtung L kann dabei zugleich auch die Übertragung der Meß- und Fehlsichtigkeitsdaten an die Behandlungsvorrichtung 1 erfolgen.

Es ist vorzugsweise durch geeignete Mittel sichergestellt, daß die Planungseinrichtung P immer den zum Patienten 4 gehörenden Steuerdatensatz erzeugt, und eine irrtümliche Verwendung eines falschen Steuerdatensatzes für einen Patienten 4 ist so gut wie ausgeschlossen.

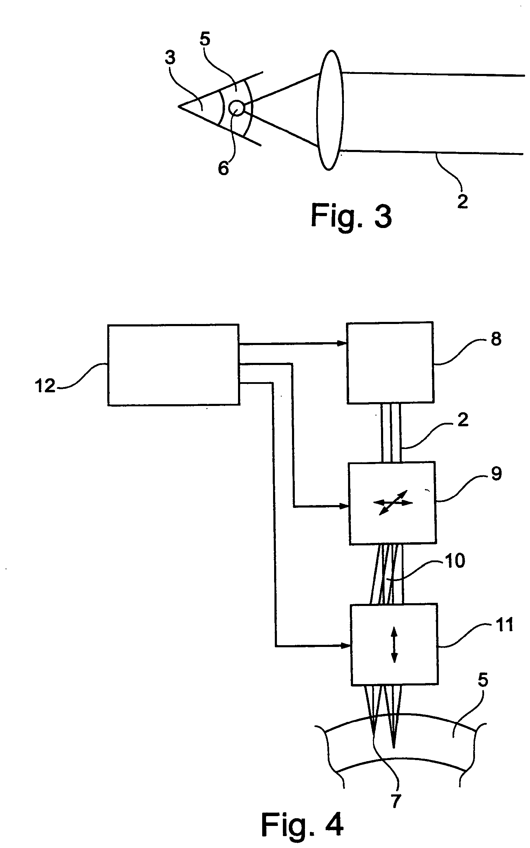

Die Wirkungsweise des Laserstrahls 2 ist in Figur 3 schematisch angedeutet. Der Behandlungs-Laserstrahl 2 wird mittels einer nicht näher bezeichneten Optik in die Hornhaut 5 des Auges 6 fokussiert. Dadurch entsteht in der Hornhaut 5 ein Fokus, der einen Spot 6 überdeckt und in dem die Laserstrahlungsenergiedichte so hoch ist, daß in Kombination mit der Pulslänge ein nicht-linearer Effekt im Auge auftritt. Beispielsweise kann jeder Puls der gepulsten Laserstrahlung 2 am jeweiligen Spot 6 einen optischen Durchbruch in der Augenhornhaut 5 erzeugen, welcher wiederum eine in Figur 3 schematisch angedeutete Plasmablase initiiert. Dadurch wird dieses Laserpulses in der Hornhaut 5 Gewebe getrennt. Bei Entstehung einer Plasmablase umfaßt die Gewebeschichttrennung ein größeres Gebiet, als den Spot 6, welchen der Fokus der Laserstrahlung 2 überdeckt, obwohl die Bedingungen zur Erzeugung des Durchbruches nur im Fokus erreicht werden. Damit von jedem Laserpuls ein optischer Durchbruch von jedem Laserpuls erzeugt wird, muß die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt.

Alternativ kann ein gewebetrennender Effekt durch die gepulste Laserstrahlung auch dadurch erzeugt werden, daß mehrere Laserstrahlungspulse im einen Bereich abgegeben werden, wobei sich für mehrere Laserstrahlungspulse die Spots 6 überlappen. Es wirken dann mehrere Laserstrahlungspulse zusammen, um einen gewebetrennenden Effekt zu erreichen.

Die Art der Gewebetrennung, die das Behandlungsgerät 1 einsetzt, ist jedoch für die nachfolgende Beschreibung nicht weiter relevant, auch wenn in dieser Beschreibung gepulste Behandlungs-Laserstrahlung 2 geschildert wird. Beispielsweise kann ein Behandlungsgerät 1 verwendet werden, wie sie in der WO 2004/032810 A2 beschrieben ist. Eine Vielzahl von Laserpulsfoki bildet im Gewebe eine Schnittfläche aus, deren Form vom Muster abhängt, mit dem die Laserpulsfoki im Gewebe angeordnet sind/werden. Das Muster gibt Zielpunkte für die Fokuslage vor, an denen ein oder mehrere Laserpuls(e) abgegeben wird(werden), und definiert die Form und Lage der Schnittfläche.

Um nun eine Fehlsichtigkeitskorrektur auszuführen, wird mittels der gepulsten Laserstrahlung aus einem Gebiet innerhalb der Hornhaut 5 Material entfernt, indem dort Gewebeschichten getrennt werden, die das Material isolieren und dann eine Materialentnahme ermöglichen. Die Materialentfernung bewirkt eine Volumenänderung in der Hornhaut, welche eine Änderung der optischen Abbildungswirkung der Hornhaut 5 zur Folge hat, die genau so bemessen ist, daß damit die zuvor ermittelte Fehlsichtigkeit möglichst korrigiert ist/wird. Zur Isolierung des zu entfernenden Volumens wird der Fokus der Laserstrahlung 2 auf Zielpunkte in der Hornhaut 5 gerichtet, in der Regel in einem Bereich, der unterhalb des Epithels und der Bowman'schen Membran sowie oberhalb der Decemetschen Membran und des Endothels liegt. Das Behandlungsgerät 1 weist dazu einen Mechanismus zum Verstellen der Lage des Fokus der Laserstrahlung 2 in der Hornhaut 5 auf. Dies ist schematisch in Figur 3 gezeigt.

In Figur 4 sind Elemente des Behandlungsgeräts 1 nur insoweit eingetragen, als sie zum Verständnis der Fokusverstellung erforderlich sind. Die Laserstrahlung 2 wird, wie bereits erwähnt, in einem Fokus 7 in der Hornhaut 5 gebündelt, und die Lage des Fokus 7 in der Hornhaut wird verstellt, so daß zur Schnittflächenerzeugung an verschiedenen Stellen fokussiert Energie aus Laserstrahlungspulsen in das Gewebe der Hornhaut 3 eintragen wird. Die Laserstrahlung 2 wird von einem Laser 8 als gepulste Strahlung bereitgestellt. Ein xy-Scanner 9, der in einer Variante durch zwei im wesentlichen orthogonal ablenkende Galvanometerspiegel realisiert ist, lenkt den vom Laser 8 kommenden Laserstrahl zweidimensional ab, so daß nach dem xy-Scanner 9 ein abgelenkter Laserstrahl 10 vorliegt. Der xy-Scanner 9 bewirkt somit eine Verstellung der Lage des Fokus 7 im wesentlichen senkrecht zur Haupteinfallsrichtung der Laserstrahlung 2 in die Hornhaut 5. Zur Verstellung der Tiefenlage ist neben dem xy-Scanner 9 ein z-Scanner 11 vorgesehen, der beispielsweise als verstellbares Teleskop ausgebildet ist. Der z-Scanner 11 sorgt dafür, daß die z-Position der Lage des Fokus 7, d.h. dessen Position auf der optischen Achse des Einfalls verändert wird. Der z-Scanner 11 kann dem xy-Scanner 9 nach- oder vorgeordnet sein. Die nachfolgend mit x, y, z bezeichneten Koordinaten beziehen sich also auf die Ablenkung der Lage des Fokus 7.

Für das Funktionsprinzip des Behandlungsgerätes 1 ist die Zuordnung der einzelnen Koordinaten zu den Raumrichtungen nicht wesentlich, der einfacheren Beschreibung halber ist jedoch nachfolgend mit z immer die Koordinate entlang der optischen Achse des Einfalls der Laserstrahlung 2 bezeichnet, und x sowie y bezeichnen zwei zueinander orthogonale Koordinaten in einer Ebene senkrecht zur Einfallsrichtung des Laserstrahls. Dem Fachmann ist natürlich bekannt, daß eine dreidimensionale Beschreibung der Lage des Fokus 7 in der Hornhaut 5 auch durch andere Koordinatensysteme erfolgen kann, insbesondere muß es sich nicht um ein rechtwinkliges Koordinatensystem handeln. Daß der xy-Scanner 9 um zueinander rechtwinklige Achsen ablenkt, ist also nicht zwingend, vielmehr kann jeder Scanner verwendet werden, der in der Lage ist, den Fokus 7 in einer Ebene zu verstellen, in der die Einfallsachse der optischen Strahlung nicht liegt. Somit sind auch schiefwinklige Koordinatensysteme möglich.

Weiter können auch nicht-kartesische Koordinatensysteme zur Beschreibung bzw. Steuerung der Lage des Fokus 7 verwendet werden, wie dies nachfolgend auch noch erläutert wird. Beispiele für solche Koordinatensysteme sind Kugelkoordinaten (auch als sphärische Koordinaten bezeichnet) sowie zylindrische Koordinaten.

Zur Steuerung der Lage des Fokus 7 werden der xy-Scanner 9 sowie der z-Scanner 11, die gemeinsam ein konkretes Beispiel einer dreidimensionalen Fokusverstelleinrichtung realisieren, von einem Steuergerät 12 über nicht näher bezeichnete Leitungen angesteuert. Gleiches gilt für den Laser 8. Das Steuergerät 12 sorgt für einen geeignet synchronen Betrieb des Lasers 8 sowie der dreidimensionalen Fokusverstelleinrichtung, exemplarisch realisiert durch den xy-Scanner 9 sowie den z-Scanner 11, so daß die Lage des Fokus 7 in der Hornhaut 5 so verstellt wird, daß letztendlich ein Material bestimmten Volumens isoliert wird, wobei die spätere Volumenentfernung eine gewünschte Fehlsichtigkeitskorrektur bewirkt.

Das Steuergerät 12 arbeitet nach vorgegebenen Steuerdaten, welche die Zielpunkte für die Fokusverstellung vorgeben. Die Steuerdaten sind in der Regel in einem Steuerdatensatz zusammengefaßt. Dieser gibt in einer Ausführungsform die Koordinaten der Zielpunkte als Muster vor, wobei die Reihenfolge der Zielpunkte im Steuerdatensatz die Aneinanderreihung der Fokuslagen und damit letztlich eine Bahnkurve (hier auch verkürzt als Bahn bezeichnet) festlegt. Der Steuerdatensatz enthält in einer Ausführungsform die Zielpunkte als konkrete Stellwerte für den Fokuslagenverstellmechanismus, z.B. für den xy-Scanner 9 und den z-Scanner 11. Zur Vorbereitung des augenchirurgischen Verfahrens, also bevor das eigentliche Operationsverfahren ausgeführt werden kann, werden die Zielpunkte und vorzugsweise auch deren Reihenfolge im Muster bestimmt. Es muß eine Vorplanung des operativen Eingriffes dahingehend erfolgen, daß die Steuerdaten für das Behandlungsgerät 1 ermittelt werden, deren Anwendung dann eine für den Patienten 4 optimale Fehlsichtigkeitskorrektur erreicht.

Zuerst gilt es das aus in der der Hornhaut 5 zu isolierende und später zu entfernende Volumen festzulegen. Wie bereits anhand Figur 2 geschildert, bedarf es dazu einer Feststellung des Korrekturbedarfs.

Hinsichtlich der in dieser Beschreibung verwendeten Nomenklatur sei angemerkt, daß durch die Anfügung eines Sterns an Größen verdeutlicht wird, daß es sich um Größen handelt, die nach einer Korrektur erhalten werden. Unter der gerechtfertigten Annahme, daß eine Dickenänderung der Hornhaut 5 im wesentlichen den Krümmungsradius der Luft zugewandten Hornhaut-Vorderseite 15 modifiziert, nicht aber den Krümmungsradius der dem Augeninneren zuliegenden Hornhautrückseite 16, wird durch die Volumenentfernung der Krümmungsradius $R_{CV}$ der Hornhautvorderseite 15 modifiziert. Die um das Volumen verminderte Hornhaut 5 mit veränderter Hornhautoberfläche 15* hat aufgrund der modifizierten Vorderseitenkrümmung eine entsprechend geänderte Abbildungswirkung, so daß ein korrigierter Fokus auf der Netzhaut 14 liegt.

Zur Bestimmung des Musters der Zielpunkte wird deshalb die zu erreichende Krümmung $R^*_{CV}$ der modifizierten Hornhautvorderfläche 15* ermittelt.

Mit dem Wert $B_{COR}$ wird nun die Krümmung der modifizierten Hornhautvorderfläche 15* wie folgt eingestellt:

$$(1) \qquad R_{CV}^*(r,\varphi) = 1 / ( (1/R_{CV}(r,\varphi)) + B_{COR}(r,\varphi)/ (n_c-1) ) + F,$$

In Gleichung (1) bezeichnet $n_c$ die Brechkraft des Materials der Hornhaut. Der entsprechende Wert liegt üblicherweise bei 1,376; $B_{COR}$ bezeichnet eine Brechkraftänderung, die zur Fehlsichtigkeitskorrektur nötig ist. $B_{COR}$ ist radial abhängig. Unter radialer Abhängigkeit wird dabei verstanden, daß es zwei Werte r1 und r2 für den Radius r gibt, für die die Brechkraftänderung bei allen Winkeln $\varphi$ unterschiedliche Werte hat.

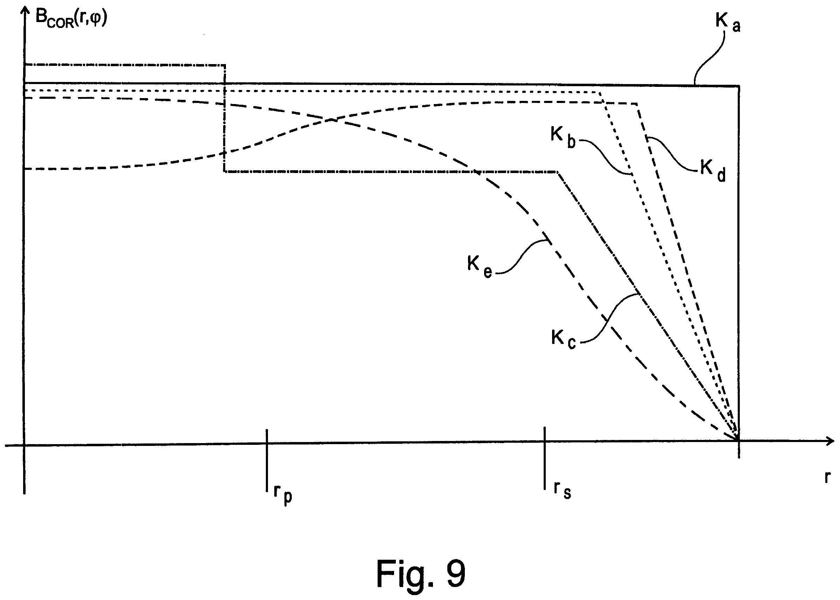

Beispiele für mögliche Verläufe der Brechkraftänderung sind in Figur 9 exemplarisch gezeigt, die die Funktion $B_{COR}$ in verschiedenen Beispielkurven Ka bis Ke als Funktion des Radius r zeigt.

Ka ist die herkömmliche Brechzahl einer Brille des Standes der Technik gemäß DE 102006053120 A1, in der Darstellung der Figur 9 jedoch bereits auf die Ebene des Hornhautscheitels bezogen. Für diesen Bezug gibt es im genannten Stand der Technik aber keinen Anlaß. Er wurde hier nur zur besseren Vergleichbarkeit mit den erfindungsgemäßen beispielhaften Verläufen Kb bis Ke eingetragen. Der Verlauf Kb verläuft bis zu einem Radius, der jenseits eines Radius $r_s$ liegt, konstant und fällt dann ab. Der Radius $r_s$ ist dabei der skotopische Pupillenradius, also der Pupillenradius, der sich beim Dunkelsehen einstellt. Die Brechkraftänderung gemäß Kurve Kc ist bis zum Wert $r_s$ stückweise konstant, wobei unterhalb eines Radius $r_p$, der dem photopischen Pupillenradius entspricht, ein Sprung von einem höheren Wert auf einen niedrigeren Wert erfolgt. Eine solche Variation der Brechkraftkorrektur über dem Pupillenquerschnitt ist bei Altersweitsichtigkeit besonders vorteilhaft. Dort findet Sehen im Nahbereich üblicherweise bei guter Beleuchtung statt, z. B. beim Lesen. Aufgrund der guten Beleuchtung ist die Pupille dann in der Regel auf den photopischen Pupillenradius verengt. Der dann nötige Brechkraftkorrekturwert stellt eine optimale Anpassung an das Nahsehen ein, z. B. einen optimalen Sehabstand von etwa 25 bis 70 cm. Für den anderen Extremfall, nämlich dem Dunkelsehen, das üblicherweise mit einer Sicht in die Ferne verknüpft ist (z. B. bei nächtlichen Autofahrten) ist dagegen die Pupille maximal geöffnet. Dann wirken auch Bereiche der Pupille bei der optischen Abbildung mit, die einen anderen (z. B. niedrigeren) Wert für die Brechkraftkorrektur haben. Das menschliche Gehirn ist in der Lage, eine derart mit optischen Fehlern behaftete Abbildung (unterschiedliche Fokuslage für das Zentrum der Pupille und Randbereiche der Pupille) bei der optischen Wahrnehmung zu korrigieren. Die in den Kurven Kc oder Kd gezeigten Brechkraftkorrekturverläufe erlauben es also durch bewußtes in Kauf nehmen eines Abbildungsfehlers den Tiefenschärfebereich zu vergrößern, da der Abbildungsfehler vom Gehirn kompensiert wird.

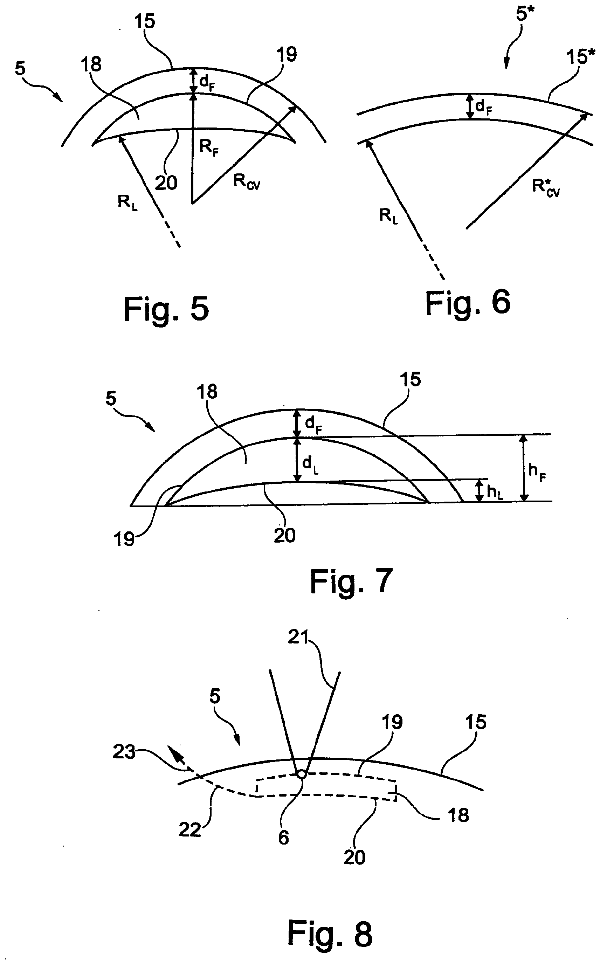

Ab dem Pupillenradius $r_s$ fällt die Brechkraftkorrektur dann weiter ab. Der nichtstufenförmige Abfall der Brechwertkorrektur auf den Wert Null ist aus anatomischer Sicht vorteilhaft. Er erlaubt am Rand des korrigierten Bereiches, d. h. am Rande des zu entfernenden Volumens, eine Anpassung des korrigierten Hornhautvorderseitenradius, welcher sich aufgrund der Korrektur einstellt, an den ursprünglichen Hornhautkrümmungsradius, d. h. den prä-operativen Radius. Bezogen auf die Darstellung der Figur 5 heißt dies, daß im Randbereich des zu entfernenden Volumens, an dem in der Darstellung der Figur 5 die Radien $R_F$ und $R_L$ zusammenlaufen, eine Angleichung dieser Radien erfolgt. Dadurch ist an der Hornhautvorderfläche nach der Korrektur der Übergang vom neuen Hornhautvorderseitenradius $R^*_{CV}$, der in dem Bereich vorliegt, in dem das Volumen 18 entnommen wurde, an den ursprünglichen Hornhautkrümmungsradius $R_{CV}$ vergleichsmäßig ist. Dadurch ist die optische Korrektur insgesamt besser, was erst durch die radial variierende Brechkraftkorrektur erzielbar ist.

Der Abfall der Brechkraftkorrektur auf den Wert Null erfolgt vorzugsweise in einem Bereich außerhalb des dunkel angepaßten Pupillenradius, also in einem für das Sehen nicht weiter relevanten Bereichs der Augenhornhaut.

Einen ähnlichen Verlauf zeigt die Kurve Kd, allerdings findet hier ein gleitender Übergang vom ersten Wert der Brechkraftänderung unterhalb $r_p$, auf den zweiten Wert, der bei $r_s$ vorliegt, statt. Zudem ist exemplarisch der erste Wert hier niedriger als der zweite Wert. Dies kann natürlich auch für die Kurve Kc so verwendet werden, je nach gewünschtem Korrekturbedarf. Einen gleitenden Verlauf, der kontinuierlich abnimmt, zeigt Kurve Ke.

Die anhand Figur 9 geschilderten örtlich abhängigen Brechkraftänderungen mit radialer Abhängigkeit sind Beispiele für eine Brechkraftänderung, die bei der Bestimmung des in der Operation zu entfernenden Volumens verwendet wird.

Der Faktor F drückt die optische Wirkung der Dickenänderung aus, welche die Hornhaut durch den operativen Eingriff erfährt und kann in erster Näherung als konstanter Faktor angesehen werden, der z. B. experimentell zuvor bestimmt werden kann. Für eine hochgenaue Korrektur kann der Faktor gemäß folgender Gleichung errechnet werden:

$$(2) \qquad F = (1 - 1/n_c) \cdot \Delta z(r=0,\varphi)$$

$\Delta z(r = 0, \varphi)$ ist dabei die zentrale Dicke des zu entfernenden Volumens.

Für eine genaue Bestimmung erfolgt eine Berechnung von $R_{CV}^*$ iterativ, indem bei der i-ten Berechnung aus der Differenz $1/R_{CV}^*(r=0,\varphi) - 1/R_{CV}(r=0,\varphi)$ auf die Größe $\Delta z(r=0,\varphi)$ geschlossen wird und das entsprechende daraus erhaltene Ergebnis für die Dickenänderung bei der (i+1)-ten Berechnung von $R^*_{CV}$ angewendet wird. Dies kann man so lange durchführen, bis ein Abbruchkriterium erfüllt wird, beispielsweise wenn die Differenz des Ergebnisses für die Dickenänderung bei zwei aufeinanderfolgenden Iterationsschritten unter einer entsprechend festgelegten Grenze liegt. Diese Grenze kann beispielsweise über eine konstante Differenz festgelegt werden, die einer für die Behandlung angemessene Genauigkeit der Refraktionskorrektur entspricht.

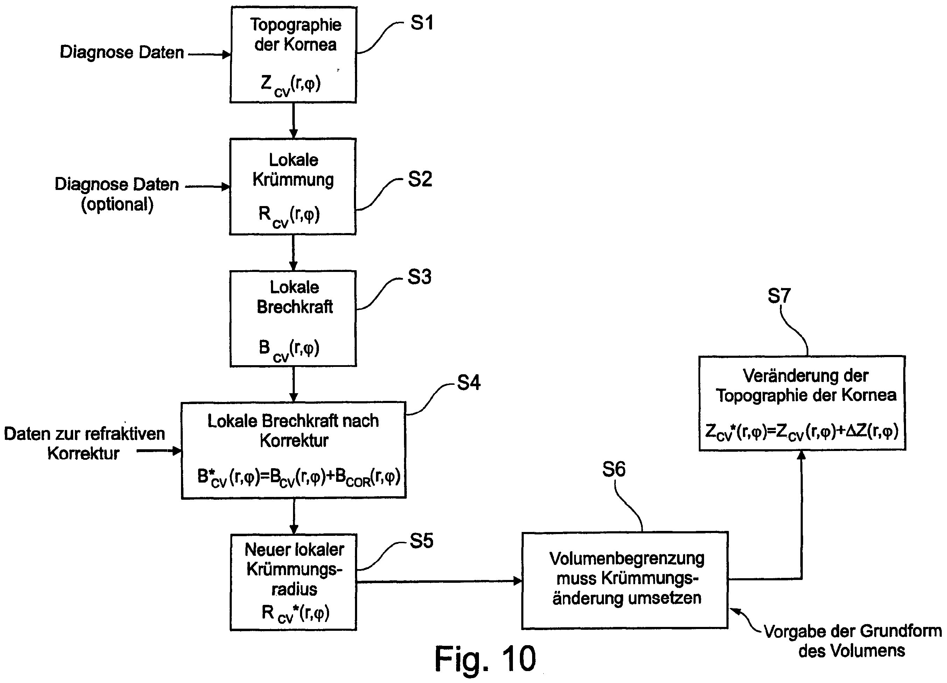

Allgemein kann dabei das in Figur 10 dargestellte Verfahren ausgeführt werden. In einem Schritt S1 wird aus Diagnosedaten die Topographie der Kornea berechnet, wie bereits eingangs im allgemeinen Teil der Beschreibung erwähnt wurde. Aus dieser Topographie wird der radiale Krümmungsverlauf der Hornhautvorderseite 15 ermittelt. Diese Ermittlung kann anstatt der Topographiedaten aus Schritt S1 auch direkt aus Diagnosedaten vorgenommen werden, so daß Schritt S2 entweder dem Schritt S1 nachgeordnet ist, oder direkt Diagnosedaten verwertet, wie Figur 10 durch die Anfügung "(optional)" verdeutlicht. Schritt S1 ist also optional.

Typische Diagnosedaten für den Schritt S2 sind Meßdaten, welche die ortsabhängige Krümmung der Kornea beschreiben, beispielsweise die Topographiedaten von Hornhaut-Topographen (Scheimpflug, Placido-Projektoren) oder einfache Keratometer, welche nur die mittlere Krümmung der Kornea auf dem stärksten und flachsten Meridian bestimmen. Es ist ebenfalls möglich, daß die globale Krümmung der Kornea als manueller Eingabeparameter oder daß sogar ein fester Wert verwendet wird, wenn keine spezielle Anpassung notwendig erscheint.

In einem Schritt S3 wird dann die lokale Brechkraft der Kornea ermittelt.

Aus Daten der gewünschten refraktiven Korrektur wird in einem Schritt S4 die erforderliche lokale Brechkraftänderung $B_{COR}$ und mit dieser aus der lokalen Brechkraft die nach der Korrektur erwünschte lokale Brechkraft bestimmt. Die gewünschte refraktive Korrektur kann dabei direkt als ortsabhängige Brechkraft eingegeben werden oder wie eingangs erwähnt äquivalent auch in Form einer allgemeinen Wellenfrontänderung, die dann beispielsweise in Form von Zernike-Koefizienten repräsentiert sein kann.

Wenn die ortsabhängige Brechkraft verwendet wird, so gelingt damit eine besonders anschauliche Darstellung insbesondere dann, wenn, anstatt einer lokalen Brechkraft an einem bestimmten Punkt, der Mittelwert der Brechkraft über ein Ringsegment, insbesondere über eine Kreisfläche, angezeigt und eingegeben wird.

Es ist ebenfalls möglich, Diagnosedaten über die präoperative Wellenfront einzubeziehen, um die zu realisierende Änderungen der Wellenfront abzuleiten, welche wiederum zu einer bestimmten Zielwellenfront führt. Weiter sind Mischformen möglich, bei denen die Daten der subjektiv bestimmten Refraktionswerte Sphäre/Zylinder/Achse mit den Daten der Wellenfrontmessung kombiniert werden.

Aus dieser ergibt sich in Schnitt S5 der neue lokale Krümmungsradius $R^*_{CV}(r, \varphi)$. Anstelle der Berechnung der lokalen Brechkraft $B_{CV}$ in Schritt S3 kann auch direkt mit der lokalen Krümmung $R_{CV}$ aus Schritt S2 gerechnet werden, wenn die obige Gleichung (1) verwendet wird. Hierbei ist ganz grundlegend darauf hinzuweisen, daß Brechkraft und Krümmungsradius mit einer einfachen Gleichung ineinander überführt werden können. Es gilt: $B = (n_C-1)/R$, wobei B die Brechkraft und R der für diese Brechkraft zugeordnete Radius ist. Es ist also im Rahmen der Erfindung jederzeit möglich, zwischen Radius-Betrachtungsweise und Brechkraft-Betrachtungsweise bzw. -Darstellung zu wechseln. Die bei der Ermittlung der Steuerdaten bei Brechkraft-Darstellungen zu verwendende Gleichung lautet:

$$B^*_{CV}(r,\varphi) = \cfrac{1}{\cfrac{1}{B_{CV}(r,\varphi) + B_{COR}(r,\varphi)} + \cfrac{F}{(n_C - 1)}}$$

Soweit hier von dem Radius der Hornhautvorderfläche gesprochen wird, kann völlig analog auch die Brechkraft verwendet werden, so daß alle hier im Zusammenhang mit dem Radius der Hornhautvorderfläche gegebene Erläuterungen selbstverständlich analog auch für die Brechkraft-Darstellung bzw. -Sichtweise gelten, wenn R durch B gemäß dem genannten Zusammenhang ersetzt wird.

Für das Volumen, dessen Entfernung die obige Krümmungsänderung der Hornhautvorderfläche 15 bewirkt, wird nun in einem Schritt S6 die das Volumen isolierende Grenzfläche festgelegt. Dabei ist zu berücksichtigen, welche Grundform das Volumen haben soll.

In einer ersten Variante wird mittels dem Fachmann bekannter numerischer Methoden eine Freifläche definiert, die das Volumen umschreibt, dessen Entfernung die Krümmungsänderung bewirkt. Dazu wird entlang der z-Achse die Volumendicke ermittelt, die zur gewünschten Krümmungsmodifikation nötig ist. Daraus ergibt sich das Volumen als Funktion von r, $\varphi$ (in Zylinderkoordinaten) und daraus wiederum dessen Grenzfläche.

Eine analytische Rechnung liefert hingegen die folgende, bereits in der DE 102006053120 A1 angesprochene Variante, bei der die Grenzfläche des Volumens im wesentlichen durch zwei Teilflächen aufgebaut wird, eine zur Hornhautoberfläche 15 hinliegende anteriore Teilfläche und eine gegenüberliegende posteriore Teilfläche. Die entsprechenden Verhältnisse zeigt Figur 5. Das Volumen 18 ist zur Hornhautvorderfläche 15 hin durch eine anteriore Schnittfläche 19 begrenzt, die in konstantem Abstand $d_F$ unter der Hornhautvorderfläche 15 liegt. Diese anteriore Schnittfläche 19 wird in Analogie zur Laserkeratomen auch als Flapfläche 19 bezeichnet, da sie dort dazu dient, in Kombination mit einem Öffnungsschnitt zum Rand hin die Augenhornhaut 5 eine Lamelle in Form eines "Flap" von der darunterliegenden Hornhaut 5 abheben zu können. Diese Art der Entnahme des zuvor isolierten Volumens 18 ist natürlich auch hier möglich.

Die anteriore Schnittfläche 19 ist bevorzugt sphärisch, da dann für sie ein Krümmungsradius angegeben werden kann, der um die Lamellendicke $d_F$ geringer ist als der Krümmungsradius $R_{CV}$.

Posterior ist das Volumen 18, das aus der Hornhaut 5 entfernt werden soll, durch eine posteriore Schnittfläche 20 begrenzt, die schon grundsätzlich nicht zur Hornhautvorderfläche 15 in konstantem Abstand sein kann. Die posteriore Schnittfläche 20 wird deshalb so ausgebildet sein, daß das Volumen 18 in Form eines Lentikels vorliegt, weshalb die posteriore Schnittfläche 20 auch als Lentikelfläche bezeichnet wird. In Figur 5 ist sie exemplarisch als ebenfalls sphärische Fläche mit einem Krümmungsradius $R_L$ eingezeichnet, wobei natürlich das Zentrum dieser Krümmung nicht mit dem Krümmungszentrum der in Figur 5 ebenfalls sphärischen Hornhautvorderfläche 15 zusammenfällt. Am Rand werden die beiden Flächen 19, 20 vorzugsweise durch eine Lentikelrandfläche verbunden, um das zu entnehmende Volumen vollständig zu umgrenzen und zugleich eine Mindestdicke am Rand zu gewährleisten.

Figur 6 zeigt die Verhältnisse nach Entfernung des Volumens 18. Der Radius der modifizierten Hornhautvorderfläche 15* beträgt nun $R_{CV}$* und kann beispielsweise gemäß den zuvor beschriebenen Gleichungen berechnet werden. Die Dicke $d_L = \Delta z(r=0,\varphi)$ des entnommenen Volumens 18 ist dabei maßgeblich für die Radiusänderung, wie Figur 7 verdeutlicht. In dieser Figur ist vereinfacht die Lentikelfläche sphärisch. Folglich sind als weitere Größen noch die Höhe $h_F$ der durch die Flapfläche 19 definierten Kugelkappe, die Höhe $h_L$ der durch die Lentikelfläche 20 definierten Kugelkappe sowie die Dicke $d_L$ des zu entfernenden Volumens 18 eingezeichnet.

Die Lentikelfläche 20 legt aufgrund des konstanten Abstandes zwischen Hornhautvorderfläche 15 und Flapfläche 19 den Krümmungsverlauf der Hornhautvorderfläche 15* nach Entfernung des Volumens 18 fest.

Soll der Faktor F bei der Berechnung berücksichtigt werden, wird in Schritt S7 noch die Veränderung der Topographie der Kornea berücksichtigt, d.h. die aktuelle Mittendicke berechnet. Mit dem sich daraus ergebenden Wert für den Faktor F können dann die Schritte S4 bis S6 oder S5 bis S6 nochmals oder mehrmals in Form einer Iteration durchlaufen werden.

Die in den Figuren gezeigte Ausbildung des Volumens 18 als durch eine Flapfläche 19 mit konstantem Abstand zur Hornhautvorderfläche 15 sowie eine Lentikelfläche 20 begrenzt, ist nur eine Variante zur Begrenzung des Volumens 18. Sie hat jedoch den Vorteil, daß die optische Korrektur wesentlich nur durch eine Fläche (die Lentikelfläche 20) festgelegt wird, so daß die analytische Beschreibung der anderen Teilfläche der Grenzfläche einfach ist.

Weiter sind optimale Sicherheitsmargen hinsichtlich des Abstandes des Volumens zur Hornhautvorderfläche 15 und Hornhautrückfläche 16 gegeben. Die Restdicke $d_F$ zwischen Flapfläche 19 und Hornhautvorderfläche 15 kann konstant auf einen Wert von beispielsweise 50 bis 200 μm eingestellt werden. Insbesondere kann sie so gewählt sein, daß das schmerzempfindliche Epithel in der Lamelle verbleibt, die durch die Flapfläche 19 unter der Hornhautvorderfläche 15 gebildet ist. Auch steht die Ausbildung der sphärischen Flapfläche 19 in Kontinuität mit bisherigen Keratometerschnitten, was für die Akzeptanz der Methode vorteilhaft ist.

Nach Erzeugen der Schnittflächen 19 und 20 wird dann das derart isolierte Volumen 18 aus der Hornhaut 5 entfernt. Dies ist schematisch in Figur 8 dargestellt, die zudem verdeutlicht, daß die Schnittflächen 19 und 20 durch Einwirkung des in einem Fokuskegel 21 einfallenden Behandlungslaserstrahls erzeugt werden, beispielsweise durch Aneinanderreihung von Plasmablasen, so daß in einer bevorzugten Ausführungsform die Flapfläche 19 und die Lentikelfläche 20 durch geeignete dreidimensionale Verstellung der Fokuslage der gepulsten Laserstrahlung 2 erzeugt werden.

Alternativ kann in einer vereinfachten Ausführungsform aber auch lediglich die Flapfläche 19 durch Zielpunkte, die die gekrümmte Schnittfläche 19 in konstantem Abstand zu Hornhautvorderfläche 15 definieren, mittels gepulster Laserstrahlung gebildet werden und die Entfernung des Volumens 18 erfolgt durch Laserablation, beispielsweise durch Verwendung eines Excimers-Laserstrahls. Hierzu kann die Lentikel-Fläche 20 als Grenzfläche des Abtrages definiert werden, auch wenn das nicht zwingend erforderlich ist. Das Behandlungsgerät 1 arbeitet dann wie ein bekanntes Laserkeratom, allerdings wird die Schnittfläche 19 an gekrümmter Hornhaut erzeugt. Die vorangehend bzw. nachfolgend beschriebenen Merkmale sind auch in solchen Varianten möglich, insbesondere was die Bestimmung der Begrenzungsfläche, deren geometrische Definition und die Ermittlung von Steuerparametern angeht.

Erzeugt man sowohl die Lentikelfläche 20 als auch die Flapfläche 19 mittels gepulster Laserstrahlung, ist es zweckmäßig, die Lentikelfläche 20 vor der Flap-Fläche 19 auszubilden, da das optische Ergebnis bei der Lentikelfläche 20 besser (wenn nicht überhaupt erst zu erreichen) ist, wenn oberhalb der Lentikelfläche 20 noch keine Veränderung der Hornhaut 5 eintrat.

Wie bereits im allgemeinen Teil der Beschreibung erläutert, ist für die hier beschriebene Fehlsichtigkeitskorrektur sowohl die exakte Positionierung der Augenhornhaut als auch genaue Kenntnis über die Krümmung der Augenhornhaut wesentlich. Nur bei Kenntnis dieser Krümmung kann beispielsweise der beschriebene Flapschnitt erzeugt werden und die gewünschte Korrekturfläche als Schnittfläche in Form der Lentikelfläche erreicht werden.

Es wird deshalb ein Kontaktglas verwendet, das eine gekrümmte Kontaktfläche hat. An diese wird die Hornhaut angepreßt. Dieser Vorgang führt, wie bereits eingangs geschildert, zu einer Deformation der Hornhaut.

Hat man eine Korrekturfläche ermittelt, die im Auge als Schnittfläche erzeugt werden soll, so betrifft diese Korrekturfläche natürlich die undeformierte Augenhornhaut. Die Schnittfläche wird allerdings in der deformierten Augenhornhaut erzeugt, so daß die Korrekturfläche bzw. die Schnittfläche vor der Erzeugung der Steuerdaten entsprechend modifiziert werden muß. Diese Modifikation stellt eine Vorverzerrung der Korrekturfläche bzw. der zu erzeugenden Schnittfläche dar, wobei die Fläche so verändert wird, daß nach der Abnahme des Kontaktglases, d. h. nach der Relaxation der Verformung auch die gewünschte Flächenform erreicht ist.

Diese Vorverzerrung der vorgegebenen Korrekturfläche erfolgt dadurch, daß die vorgegebene Korrekturfläche einer Koordinatentransformation unterzogen wird, welche die Hornhautverformung wiedergibt. Solche Koordinatentransformationen sind aus der US 2007/0293851 A1 sowie aus der DE 102008017293 A1 bekannt.

Wie oben erläutert, wird die lokal veränderliche refraktive Korrektur $B_{COR}(r,\varphi)$ dadurch erreicht, daß mittels gepulster Laserstrahlung Gewebsschichten entlang von Schnittflächen $F:=z(r,\varphi): R^2 \rightarrow R^3$ in der Kornea getrennt werden, so daß ein umschlossenes Teilvolumen entsteht, welches dann entnommen wird. Durch diesen Schritt wird die Form der Vorderfläche der Kornea gezielt so verändert, daß die gewünschte Korrektur entsteht. Dazu wird z. B. ein Behandlungsstrahl in die Hornhaut des Auges 6 fokussiert.

Die Form $F_A:=z_A(r,\varphi)$ der anterioren Schnittfläche 19 und die Form $F_P:=z_P(r,\varphi)$ der posterioren Schnittfläche 20 zu entnehmenden Volumens 18 sind wesentlich dadurch bestimmt, daß das Dickenprofil $\Delta z(r,\varphi) = z_A(r,\varphi) - z_P(r,\varphi)$ des umschnittenen Materials nach der Entnahme zu der benötigten Formveränderung der Korneavorderfläche 15 führt. Daher ist eine exakte absolute Positionierung der einzelnen Laserschüsse im Bereich von Mikrometern innerhalb der Kornea 5 des zu behandelnden Auges zwingend notwendig, um die Genauigkeiten der refraktiven Korrektur zu gewährleisten, weshalb dazu die Position der Kornea in Bezug auf das optische System der Laserstrahlung fixiert und die Hornhautvorderfläche 15 in bekannte Form gebracht wird, indem das Kontaktglas an die Kornea 5 des Auges angepreßt und z. B. mittels Unterdruck mechanisch fixiert wird (vgl. US 2008/234707 A1).

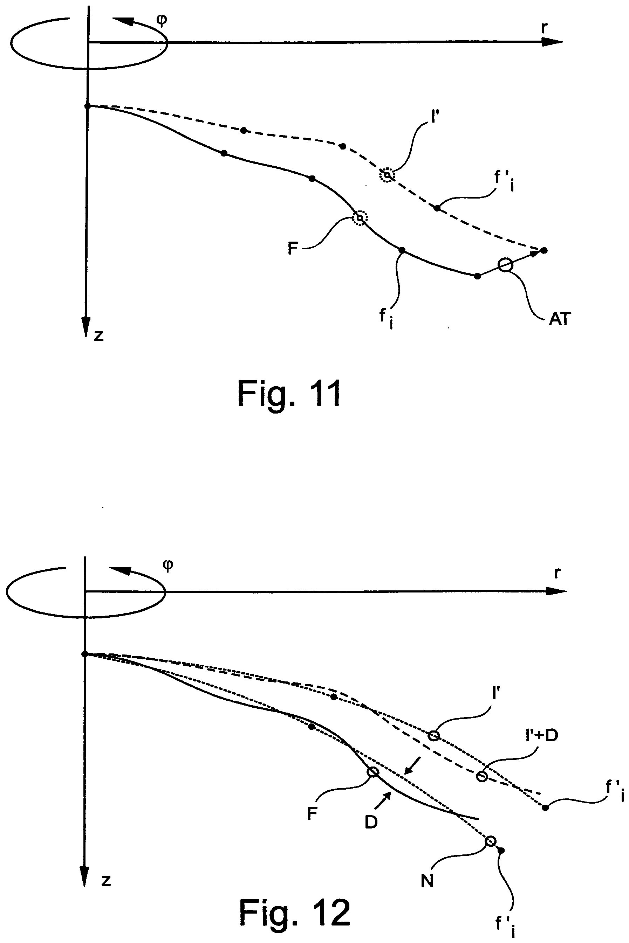

Das Kontaktglas verändert die Form der Hornhautvorderfläche. Die Deformation wird in der US 2007/0293851 anhand der Fig. 11-13 beschrieben und als Anpreßtransformation ($AT : R^3 \to R^3$) bezeichnet. Wie in den zuvor genannten Druckschriften auch, werden in dieser Beschreibung Größen, die sich auf das transformierte Koordinatensystem beziehen, durch ein Hoch, gekennzeichnet.

Für die Fehlsichtigkeitskorrektur wird eine Korrekturfläche ermittelt, die als Schnittfläche im Auge erzeugt werden soll. Gegebenenfalls kann es sich auch um mehrere Flächen handeln. Unter Korrekturfläche wird hier jede Schnittfläche verstanden, die im Auge erzeugt werden muß und die für die Refraktionsänderung im Auge wirksam ist. Die Flap-Fläche gehört z. B. dazu nicht. Bevorzugt ist jedoch die oben geschilderte Ausführungsform, welche die optische Korrektur in einer einzigen Fläche konzentriert, nämlich der Lentikelfläche. Die Ermittlung der Korrekturfläche kann dabei auf beliebige Art und Weise erfolgen, beispielsweise unter Rückgriff auf die oben erwähnten Prinzipien. Die 3-dimensionalen Schnittflächen entstehen z. B. durch die Aneinanderreihung von einzelnen Laserschüssen entlang einer Bahnkurve zu Schnittlinien und durch die Aneinanderreihung von Schnittlinien zu Schnittflächen.

Diese Korrekturfläche wird nun der Koordinatentransformation, wie nachfolgend erläutert wird, unterworfen.

Die Korrekturfläche kann bei aufwendigeren Korrekturen, insbesondere bei Korrekturen, die sich nicht auf eine Korrekturfläche begrenzen, die durch eine Sphäre oder gegebenenfalls einer Sphäre mit einem zylindrischen Anteil angegeben werden kann, große numerische Probleme bereiten, wenn zuerst die Steuerdaten erzeugt und dann der Transformation unterworfen würden. Da die Ausdehnung der räumlichen Gewebetrennung eines einzelnen Laserschusses bei einem mit gepulster Laserstrahlung arbeitenden Systems in Bereich von wenigen Quadratmikrometern liegt und die gesamte Schnittfläche im Bereich von ca. 50 mm$^2$ liegt, wären die Koordinaten von etwa 10 Millionen Punkten zu transformieren.

Um diesen Rechenaufwand zu vermeiden, wird nur eine mehrere Größenordnung kleinere Teilmenge der Punkte der Korrekturfläche bzw. Schnittfläche als Punkte $f_i$ der Fläche F ausgewählt. Die Auswahl der Punkte berücksichtigt vorzugsweise geometrische Eigenschaften der Schnittfläche F, die in der unverformten Hornhaut erzeugt werden soll. Vorzugsweise werden eventuelle Symmetrien der Schnittfläche sowie eine Invarianz der Winkelabbildung in der Anpreßtransformation (Winkel um die optische Achse) bei der Wahl der Punkte berücksichtigt.

Nachdem derart eine Teilmenge der möglichen Punkte der Schnittfläche F ausgewählt wurde, werden diese Punkte mit der Anpreßtransformation transformiert. Anschließend wird durch Interpolation eine Interpolationsfläche I' angepaßt. Diese entspricht mit hinreichender Genauigkeit der transformierten Korrekturfläche F. In der Interpolationsfläche I' werden dann die Zielpunkte und die diese enthaltende Bahn (Bahnen) festgelegt, welche in die Steuerdaten der Behandlungsvorrichtung einfließen.

Das Verfahren sieht also folgendes vor:

Steuert man die Scanvorrichtung dann so an, daß jeder einzelne Fokuspunkt zur Erzeugung der Schnittfläche auf der transformierten Schnittfläche $F':=z'(r,\varphi)$ liegt, so hat man das korrekte Teilvolumen umschlossen, da die Abnahme des Kontaktglases dann zu einer Relaxation der Hornhaut führt, die einer Rücktransformation entspricht. Es geschieht also folgendes:

1. Es werden auf der Korrekturfläche F charakteristische Punkte $f_i$ ausgewählt und in $f_i'$ transformiert. Die Punkte $f_i$ werden vorzugsweise so gewählt, daß sie die geometrischen Eigenschaften der Schnittfläche F in der unverformten Hornhaut 5 (also im natürlichen System) repräsentieren. Vorteilhaft werden dabei die Symmetrien der Schnittfläche F beachtet. Vorteilhaft ist es zudem, die Invarianz der Winkelabbildung in der Anpreßtransformation (Winkel um die optische Achse) bei der Wahl der Punkte zu berücksichtigen.

2. An die transformierten Punkte $f_i'$ wird eine Interpolationsfläche I' angepaßt. Vorzugsweise ist dabei dafür Sorge getragen, daß die Symmetrieeigenschaften der Fläche I' nur geringfügig von der Schnittfläche F im untransformierten System abweichen.

3. Vorteilhaft ist es weiter, die Anpassung der Interpolationsfläche I' an die transformierten charakteristischen Punkte $f_i'$ derart durchzuführen, daß einer der die Fläche beschreibenden Parameter die Höhe $z$ oder ein Parameter ist, der zur Höhe $z$ ein in bekannter Beziehung stehendes Substitut ist, für den also eine Darstellung wie beispielsweise $I':=r(z,\varphi)$ oder $I':=y(z,x)$ gilt. Dadurch ist es einfacher, eine Bahnkurve für die Zielpunkte aufzufinden, die möglichst schnell abgearbeitet werden kann.

Eine grafische Veranschaulichung des ersten Verfahrens ist in Figur 11 für einen bestimmten Winkel um die z-Achse angegeben. Figur 11 zeigt exemplarisch eine Schnittdarstellung durch eine Korrekturfläche F in den Koordinaten z und r. Der Winkel $\varphi$ der Zylinderkoordinaten ist eine Drehung der r-Achse um die z-Achse. Für die Fläche F werden nun Punkte $f_i$ als charakteristische Punkte ausgewählt, die anschließend mit der Anpreßtransformation AT in Punkte $f_i'$ transformiert werden. Anschließend wird die Interpolationsfläche I' durch die transformierten Punkte $f_i'$ gelegt. Diese Interpolationsfläche I' dient dann für die Bestimmung der Steuerdaten.

Das Konzept der charakteristischen Punkte $f_i$ läßt sich jedoch auch in einer vereinfachten Variante anwenden. Hierbei wird die Information über die Veränderung der Fläche $F \rightarrow F'$ im Zuge der Anpreßtransformation $AT$ nicht mehr vollständig übertragen. Es wird stattdessen im transformierten System (Kontaktglassystem) eine geeignete Näherungsfläche $N$ gewählt, die der Transformation $AT$ unterworfen wird. Dabei werden für die Näherungsfläche wiederum charakteristische Punkte $f_i$ ausgewählt und mit der Anpreßtransformation $AT$ in transformierte Punkte $f_i'$ umgesetzt. Anschließend wird durch Interpolation (gegebenenfalls durch Approximation) wiederum eine Interpolationsfläche I' gesucht, welche die transformierten Punkte $f_i'$ verbindet (oder approximiert).

Auch die Abweichung zwischen der eigentlichen Korrekturfläche F und der Näherungsfläche N wird berücksichtigt. Dazu wird im untransformierten System die Abweichung D zwischen den Flächen F und N bestimmt. Diese Abweichung D kann auf verschieden Art und Weise ermittelt werden. Zum einen ist es möglich, für alle charakteristischen Punkte $f_i$ die Abweichung D zu ermitteln. Man hat dann einen Satz von Abweichungswerten entsprechend dem Satz an charakteristischen Punkten. Alternativ ist es möglich, eine Abweichungsfunktion D zu ermitteln.

Die Abweichung D wird nun zur Korrektur der Interpolationsfläche I' verwendet. Entweder wird die Interpolationsfläche I' nach der Interpolation damit korrigiert oder es werden die transformierten charakteristischen Punkte $f_i'$ mit der Abweichung D korrigiert und danach die Interpolationsfläche I ermittelt. Letzteres ist dann zweckmäßig, wenn ein Satz an Abweichungsparametern für den Satz charakteristischer Punkte festgelegt wird. Es sind also folgende Merkmale vorgesehen bzw. möglich:

1. Die Information über die Schnittfläche $F$ im natürlichen System wird durch Ermitteln der Näherungsfläche $N$ reduziert. Dies erfolgt dadurch, daß für die Schnittfläche $F$ die Näherungsfläche $N$ bestimmt wird, beispielsweise eine Sphäre oder ein Zernicke-Polynom mit gegenüber der Schnittfläche reduzierter Ordnung.

2. Es werden für $N$ charakteristische Punkte $f_i$ bestimmt. Vorteilhaft werden dabei Symmetrien der Fläche $N$ beachtet. Vorteilhaft ist es zudem, die Invarianz der Winkelabbildung in der Anpreßtransformation (Winkel um die optische Achse) bei der Wahl der Punkte zu berücksichtigen.

3. Die Abweichung $D$ der Schnittfläche $F$ von der Näherungsfläche $N$ im natürlichen System (Augensystem) wird bestimmt.

4. Die Punkte $f_i$ *werden* in $f_{i'}$ transformiert und die Interpolationsfläche I' ermittelt.

5. Die Symmetrieeigenschaften der Interpolationsfläche I' weichen dann nur geringfügig von der Näherungsfläche $N$ im untransformierten System ab.

6. Um die Schnittfläche $F$ möglichst zu transformieren, wird I' mit der Abweichung D beaufschlagt, so daß eine Funktion entsteht, die eine gute Repräsentanz für eine transformierte Version der Schnittfläche $F$ ist.

7. Die Verwendung der Abweichung D kann dabei sowohl auf die Interpolationsfläche I' als auch auf die transformierten Punkte $f_i'$ angewendet werden, wie bereits erwähnt.

8. Vorteilhaft ist es, die Anpassung der Interpolationsfläche I' an die transformierten charakteristischen Punkte $f_i' f_i'$ derart durchzuführen, daß einer der Parameter der Funktion die Höhe z ist. Es kann natürlich auch ein dazu in bekannter Beziehung stehendes Substitut verwendet werden. Gleiches gilt für die Abweichung D.

Die Verwendung gepulster Laserstrahlung ist nicht die einzige Art und Weise, wie die operative Refraktionskorrektur ausgeführt werden kann. Die hier beschriebene Bestimmung von Steuerdaten für den Betrieb der Vorrichtung kann vielmehr für nahezu jedes Operationsverfahren verwendet werden, bei dem mittels einer Vorrichtung unter Steuerung durch Steuerdaten ein Volumen aus der Augenhornhaut entfernt oder dieser, wie im allgemeinen Teil der Beschreibung bereits erläutert, hinzugefügt wird.

**Patentansprüche**

1. Verfahren zur Erzeugung von Steuerdaten, die zur Ansteuerung einer Lasereinrichtung (L) zur operativen Fehlsichtigkeitskorrektur eines Auges (3) eines Patienten (4) ausgebildet sind, wobei im Betrieb der Lasereinrichtung (L) die Hornhaut (5) durch Anpressen ihrer Vorderfläche (15) an eine Kontaktfläche verformt ist, wobei im Verfahren eine auf die unverformte Hornhaut (5) bezogene Korrekturfläche vorgegeben ist, die zur Fehlsichtigkeitskorrektur als Schnittfläche in der Hornhaut zu erzeugen ist, und

- wobei im Verfahren die im Betrieb der Lasereinrichtung (L) vorliegende Verformung der Hornhaut (5) durch eine Transformation (AT) der Koordinaten für Punkte in der unverformten Hornhaut (5) in Koordinaten derselben Punkte in der verformten Hornhaut (5) berücksichtigt wird,

**dadurch gekennzeichnet, daß**

a) eine Teilmenge der in der Korrekturfläche (F) oder in einer daraus abgeleiteten Näherungsfläche (N) liegenden Punkten ($f_i$) ausgewählt werden und deren Koordinaten mittels der Transformation transformiert werden, um transformierte Punkte (fi') zu erhalten,
b) durch Interpolation an die transformierten Punkte (fi') eine Interpolationsfläche (I') angepaßt wird und
c) auf der Interpolationsfläche (I') liegende Zielpunkte ausgewählt und für die Erzeugung der Steuerdaten verwendet werden.

2.  Verfahren nach Anspruch 1, **dadurch gekennzeichnet, daß** vor Schritt a) die Näherungsfläche (N) aus der Korrekturfläche (F) durch einen Glättungs- oder Approximationsvorgang abgeleitet wird, und daß vor Schritt c) eine Abweichung (D) zwischen der Korrekturfläche (F) und der Näherungsfläche (N) ermittelt und die Interpolationsfläche (I') mit Hilfe dieser Abweichung (D) korrigiert wird.

3.  Verfahren nach Anspruch 1 oder 2, **dadurch gekennzeichnet, daß**

- die Steuerdaten zur Ansteuerung einer Lasereinrichtung (L) ausgebildet sind, welche durch Einstrahlen von Laserstrahlung (2) in die Hornhaut (5) des Auges (3) Hornhaut-Gewebe trennt,
- die Steuerdaten so erstellt werden, daß die Lasereinrichtung (L) bei Betrieb gemäß den Steuerdaten die Laserstrahlung (2) so abgibt, daß ein Volumen (18) in der Hornhaut (5) isoliert ist, dessen Entfernung aus der Hornhaut (5) die gewünschte Fehlsichtigkeitskorrektur bewirkt, und
- zur Ermittlung der Korrekturfläche berechnet wird

- - entweder ein Krümmungsradius $R_{CV}{}^*$, den die um das Volumen (18) verminderte Hornhaut (5) hat und der ortsabhängig ist und folgender Gleichung genügt:

$$R_{CV}{}^*(r,\varphi) = 1 / ( (1/R_{CV}(r,\varphi)) + B_{COR}(r,\varphi)/ (n_c-1) ) + F,$$

wobei $R_{CV}(r,\varphi)$ der lokale Krümmungsradius der Hornhaut (5) vor Entfernung des Volumens (18), $n_c$ die Brechkraft des Materials der Hornhaut (5), F ein Faktor ist, und $B_{COR}(r,\varphi)$ die für die gewünschte Fehlsichtigkeitskorrektur nötige, lokale Brechkraftänderung in einer im Scheitelpunkt der Hornhaut (5) liegenden Ebene ist,
- - oder eine Brechkraft $B_{CV}{}^*$, die die um das Volumen (18) verminderte Hornhaut (5) hat und die folgender Gleichung genügt:

$$B^*{}_{CV}(r,\varphi) = \cfrac{1}{\cfrac{1}{B_{CV}(r,\varphi) + B_{COR}(r,\varphi)} + \cfrac{F}{(n_C - 1)}}$$

wobei $B_{CV}(r,\varphi)$ die lokale Brechkraft der Hornhaut (5) vor Entfernung des Volumens (18), $n_c$ die Brechzahl des Materials der Hornhaut (5), F ein Faktor ist, und $B_{COR}(r,\varphi)$ die für die gewünschte Fehlsichtigkeitskorrektur nötige, lokale Brechkraftänderung in einer im Scheitelpunkt der Hornhaut (5) liegenden Ebene ist,

- wobei es mindestens zwei Radien r1 und r2 gibt, für die $B_{COR}(r=r1,\varphi) \neq B_{COR}(r=r2,\varphi)$ gilt.

4.  Verfahren nach Anspruch 3, **dadurch gekennzeichnet, daß** bei der Ermittlung der Steuerdaten für den Faktor F = (1 - 1/$n_c$) • $\Delta z(r=0,\varphi)$ verwendet wird, wobei $\Delta z(r=0,\varphi)$ die zentrale Dicke des in der Hornhaut (5) zu isolierenden und entfernenden Volumens (18) ist und der ortsabhängige Krümmungsradius $R_{CV}{}^*(r,\varphi)$ iterativ berechnet wird, indem bei jedem Iterationsschritt aus der Differenz der zentralen reziproken Krümmungsradien $1/R_{CV}{}^*(r=0,\varphi)$ und $1/R_{CV}(r=0,\varphi)$ auf die zentrale Dicke $\Delta z(r=0,\varphi)$ des Volumens (18) geschlossen und dieser Wert bei der Berechnung von $R_{CV}{}^*(r,\varphi)$ im nächsten Iterationsschritt angewendet wird.

5. Verfahren nach Anspruch 3 oder 4, **dadurch gekennzeichnet, daß** bei der Ermittlung der Steuerdaten die lokale Brechkraftänderung $B_{COR}(r,\varphi)$ so festgelegt wird, daß ein charakteristischer Radius $r_{ch}$ besteht, für den die Radialfunktion der Brechkraftänderung $B_{COR}(r,\varphi)$ stückweise konstant ist, für den also $B_{COR}(r<r_{ch},\varphi=\text{const}) = B_a \neq B_b = B_{COR}(r>r_{ch},\varphi=\text{const})$ gilt.

6. Verfahren nach Anspruch 3 oder 4, **dadurch gekennzeichnet, daß** bei der Ermittlung der Steuerdaten die lokale Brechkraftänderung $B_{COR}(r,\varphi)$ so festgelegt wird, daß ein zwei Radien $r_a$ und $r_b$ bestehen, für die die Radialfunktion der Brechkraftänderung $B_{COR}(r,\varphi)$ stückweise konstant ist, für die also $B_{COR}(r<r_a,\varphi=\text{const}) = B_a \neq B_b = B_{COR}(r>r_b,\varphi=\text{const})$ gilt, wobei die Radialfunktion der Brechkraftänderung $B_{COR}(r,\varphi)$ im Übergangsbereich zwischen $r_a$ und $r_b$ kontinuierlich von $B_a$ nach $B_b$ übergeht.

7. Verfahren nach einem der Ansprüche 3-6, **dadurch gekennzeichnet, daß** bei der Ermittlung der Steuerdaten die lokale Brechkraftänderung winkelunabhängig festgelegt wird.

8. Vorrichtung zur Erzeugung von Steuerdaten, die zur Ansteuerung einer Lasereinrichtung (L) zur operativen Fehlsichtigkeitskorrektur eines Auges (3) eines Patienten (4) ausgebildet sind, wobei im Betrieb der Lasereinrichtung (L) die Hornhaut (5) durch Anpressen ihrer Vorderfläche (15) an eine Kontaktfläche verformt ist und wobei eine auf die unverformte Hornhaut (5) bezogene Korrekturfläche vorgegeben ist, die zur Fehlsichtigkeitskorrektur als Schnittfläche in der Hornhaut zu erzeugen ist, und

   - wobei die Vorrichtung ausgebildet ist, bei der Erzeugung der Steuerdaten die im Betrieb der Lasereinrichtung (L) vorliegende Verformung der Hornhaut (5) durch eine Transformation (AT) der Koordinaten für Punkte in der unverformten Hornhaut (5) in Koordinaten derselben Punkte in der verformten Hornhaut (5) zu berücksichtigen,

   **dadurch gekennzeichnet, daß**

   a) die Vorrichtung ausgebildet ist, bei der Erzeugung der Steuerdaten eine Teilmenge der in der Korrekturfläche (F) oder in einer daraus abgeleiteten Näherungsfläche (N) liegenden Punkten ($f_i$) auszuwählen und deren Koordinaten mittels der Transformation zu transformieren, um transformierte Punkte (fi') zu erhalten,
   b) die Vorrichtung ausgebildet ist, bei der Erzeugung der Steuerdaten durch Interpolation an die transformierten Punkte (fi') eine Interpolationsfläche (I') anzupassen und
   c) die Vorrichtung ausgebildet ist, bei der Erzeugung der Steuerdaten auf der Interpolationsfläche (I') liegende Zielpunkte auszuwählen und für die Erzeugung der Steuerdaten zu verwenden.

9. Vorrichtung nach Anspruch 8, **dadurch gekennzeichnet, daß** die Vorrichtung ausgebildet ist, bei der Erzeugung der Steuerdaten vor Schritt a) die Näherungsfläche (N) aus der Korrekturfläche (F) durch einen Glättungs- oder Approximationsvorgang abzuleiten und vor Schritt c) eine Abweichung (D) zwischen der Korrekturfläche (F) und der Näherungsfläche (N) zu ermitteln und die Interpolationsfläche (I') mit Hilfe dieser Abweichung (D) zu korrigieren.

10. Vorrichtung nach Anspruch 8, **dadurch gekennzeichnet, daß**

    - die Steuerdaten zur Ansteuerung einer Lasereinrichtung (L) ausgebildet sind, welche durch Einstrahlen von Laserstrahlung (2) in die Hornhaut (5) des Auges (3) Hornhaut-Gewebe trennt,
    - die Vorrichtung (12) ausgebildet ist, die Steuerdaten so zu erstellen, daß die Lasereinrichtung (L) bei Betrieb gemäß den Steuerdaten die Laserstrahlung (2) so abgibt, daß ein Volumen (18) in der Hornhaut (5) isoliert ist, dessen Entfernung aus der Hornhaut (5) die gewünschte Fehlsichtigkeitskorrektur bewirkt, und
    - die Vorrichtung (12) ausgebildet ist, zur Ermittlung der Korrekturfläche zu berechnen

      - - entweder einen Krümmungsradius $R_{CV}{}^*$, den die um das Volumen (18) verminderte Hornhaut (5) hat und der ortsabhängig ist und folgender Gleichung genügt:

$$R_{CV}{}^*(r,\varphi) = 1 / ( (1/R_{CV}(r,\varphi)) + B_{COR}(r,\varphi)/ (n_c-1) ) + F,$$

wobei $R_{CV}(r,\varphi)$ der lokale Krümmungsradius der Hornhaut (5) vor Entfernung des Volumens (18), $n_c$ die Brechzahl des Materials der Hornhaut (5), F ein Faktor ist, und $B_{COR}(r,\varphi)$ die für die gewünschte Fehlsichtigkeitskorrektur nötige, lokale Brechkraftänderung in einer im Scheitelpunkt der Hornhaut (5) liegenden Ebene ist,

-- oder eine Breckraft $B^*_{CV}$, die die um das Volumen (18) verminderte Hornhaut (5) hat und die ortsabhängig ist und folgender Gleichung genügt:

$$B^*_{CV}(r,\varphi) = \cfrac{1}{\cfrac{1}{B_{CV}(r,\varphi) + B_{COR}(r,\varphi)} + \cfrac{F}{(n_C - 1)}}$$

wobei $B_{CV}(r,\varphi)$ die lokale Brechkraft der Hornhaut (5) vor Entfernung des Volumens (18), $n_c$ die Brechzahl des Materials der Hornhaut (5), F ein Faktor ist, und $B_{COR}(r,\varphi)$ die für die gewünschte Fehlsichtigkeitskorrektur nötige, lokale Brechkraftänderung in einer im Scheitelpunkt der Hornhaut (5) liegenden Ebene ist,

- wobei es mindestens zwei Radien r1 und r2 gibt, für die $B_{COR}(r=r1,\varphi) \neq B_{COR}(r=r2,\varphi)$ gilt.

11. Vorrichtung nach Anspruch 10, **dadurch gekennzeichnet, daß** die Vorrichtung (12) ausgebildet ist, bei der Ermittlung der Steuerdaten für den Faktor $F = (1 - 1/n_c) \cdot \Delta z(r=0,\varphi)$ zu verwenden, wobei $\Delta z(r=0,\varphi)$ die zentrale Dicke des in der Hornhaut (5) zu isolierenden und entfernenden Volumens (18) ist, und die Vorrichtung ausgebildet ist, den ortsabhängigen Krümmungsradius $R_{CV}{}^*(r,\varphi)$ iterativ zu berechnen, indem sie bei jedem Iterationsschritt aus der Differenz der zentralen reziproken Krümmungsradien $1/R_{CV}{}^*(r=0,\varphi)$ und $1/R_{CV}(r=0,\varphi)$ auf die zentrale Dicke $\Delta z(r=0,\varphi)$ des Volumens (18) schließt und diesen Wert bei der Berechnung von $R_{CV}{}^*(r,\varphi)$ im nächsten Iterationsschritt anwendet.

12. Vorrichtung nach Anspruch 10 oder 11, **dadurch gekennzeichnet, daß** sie bei der Ermittlung der Steuerdaten die lokale Brechkraftänderung $B_{COR}(r,\varphi)$ so festlegt, daß ein charakteristischer Radius $r_{ch}$ besteht, für den die Radialfunktion der Brechkraftänderung $B_{COR}(r,\varphi)$ stückweise konstant ist, für den also $B_{COR}(r<r_{ch},\varphi=const) = B_a \neq B_b$ $B_{COR}(r>r_{ch},\varphi=const)$ gilt.

13. Vorrichtung nach Anspruch 10 oder 11, **dadurch gekennzeichnet, daß** sie bei der Ermittlung der Steuerdaten die lokale Brechkraftänderung $B_{COR}(r,\varphi)$ so festlegt, daß ein zwei Radien $r_a$ und $r_b$ bestehen, für die die Radialfunktion der Brechkraftänderung $B_{COR}(r,\varphi)$ stückweise konstant ist, für die also $B_{COR}(r<r_a,\varphi=const) = B_a \neq \neq B_b = B_{COR}(r>r_b,\varphi=const)$ gilt, wobei die Radialfunktion der Brechkraftänderung $B_{COR}(r,\varphi)$ im Übergangsbereich zwischen $r_a$ und $r_b$ kontinuierlich von $B_a$ nach $B_b$ übergeht.

14. Vorrichtung nach einem der Ansprüche 10-13, **dadurch gekennzeichnet, daß** sie bei der Ermittlung der Steuerdaten die lokale Brechkraftänderung winkelunabhängig festlegt.

15. Computerprogrammprodukt mit Programmcode, der bei Ausführung auf einem Computer ein Verfahren nach einem der Ansprüche 1-7 durchführt.

**Claims**

1. A method for generating control data which are adapted to control a laser device (L) for surgical correction of defective vision of an eye (3) of a patient (4), wherein during operation of the laser device (L) a cornea (5) is deformed by pressing its front surface (15) against a contact surface, wherein in the method a correction surface is predetermined for the non-deformed cornea (5), which correction surface is to be produced as a cut surface in the cornea for correcting defective vision, and

- wherein in the method the deformation of the cornea (5) during operation of the laser device (L) is considered by a transformation (AT) of the coordinates of points in the non-deformed cornea (5) into coordinates of the same points in the deformed cornea (5),

**characterized in that**

a) a subset of the points ($f_i$) lying in the correction surface (F) or in an approximation surface (N) derived therefrom is selected and coordinates of the subset of the selected points are transformed by means of the transformation in order to obtain transformed points ($f_i'$),
b) an interpolation surface (I') is fitted to the transformed points ($f_i'$) by interpolation and
c) target points lying on the interpolation surface (I') are selected and used for generating the control data.

2. The method according to claim 1, **characterized in that** prior to step a) the approximation surface (N) is derived from the correction surface (F) by a smoothing or approximation process, and **in that** prior to step c) a deviation (D) between the correction surface (F) and the approximation surface (N) is determined and the interpolation surface (I') is corrected using this deviation (D).

3. The method according to claim 1 or 2, **characterized in that**

- the control data are adapted to control a laser device (L) which severs corneal tissue by irradiating laser radiation (2) into the cornea (5) of the eye (3),
- the control data are generated such that under operation according to the control data the laser device (L) emits the laser radiation (2) such that a volume (18) is isolated in the cornea (5), the removal of which volume from the cornea (5) effects the desired correction of defective vision, and
- to determine the correction surface, the following is calculated

- - either a radius of curvature $R_{CV}^*$ which radius the cornea (5) has without the volume (18), and which is locally varying and satisfies the equation

$$R_{CV}^*(r,\varphi) = 1 / (\, (1/R_{CV}(r,\varphi)) + B_{COR}(r,\varphi)/ (n_c\text{-}1) \,) + F,$$

wherein $R_{CV}(r,\varphi)$ is the local radius of curvature of the cornea (5) before the volume (18) is removed, $n_c$ is the refractive index of the material of the cornea (5), F is a coefficient, and $B_{COR}(r,\varphi)$ is the local change in optical refraction power defined in a plane lying in the vertex of the cornea (5) and required for the desired correction of defective vision,
- - or an optical refraction power $B_{CV}^*$ which the cornea (5) has without the volume (18), and which satisfies the equation

$$B_{CV}^*(r,\varphi) = \cfrac{1}{\cfrac{1}{B_{CV}(r,\varphi) + B_{COR}(r,\varphi)} + \cfrac{F}{(n_C - 1)}}$$

wherein $B_{CV}(r,\varphi)$ is the local optical refraction power of the cornea (5) before the volume (18) is removed, $n_c$ is the refractive index of the material of the cornea (5), F is a coefficient, and $B_{COR}(r,\varphi)$ is the local change in optical refraction power defined in a plane lying in the vertex of the cornea (5) and required for the desired correction of defective vision,

- wherein there are at least two radii, r1 and r2, for which $B_{COR}(r\text{=}r1,\varphi) \neq B_{COR}(r\text{=}r2,\varphi)$ holds true.

4. The method according to claim 3, **characterized in that** $F = (1 - 1/n_c) \cdot \Delta z(r\text{=}0,\varphi)$ is used for the coefficient F when generating the control data, wherein $\Delta z(r\text{=}0,\varphi)$ is the central thickness of the volume (18) to be isolated in the cornea (5) and to be removed and the local radius of curvature $R_{CV}^*(r,\varphi)$ is iteratively calculated, wherein in each iteration step the central thickness $\cdot \Delta z(r\text{=}0,\varphi)$ of the volume (18) is determined from the difference between the central reciprocal radii of curvature $1/R_{CV}^*(r\text{=}0,\varphi)$ and $1/R_{CV}(r\text{=}0,\varphi)$, and this value is used when calculating $R_{CV}^*(r,\varphi)$ in the next iteration step.

5. The method according to claim 3 or 4, **characterized in that**, when generating the control data, the local change in optical refraction power $B_{COR}(r,\varphi)$ is defined such that there is a characteristic radius $r_{ch}$ for which a radial function of the change in optical refraction power $B_{COR}(r,\varphi)$ is piecewise constant, for which thus $B_{COR}(r<r_{ch},\varphi=const) = B_a \neq B_b = B_{COR}(r>r_{ch},\varphi=const)$ holds true.

6. The method according to claim 3 or 4, **characterized in that**, when generating the control data, the local change in optical refraction power $B_{COR}(r,\varphi)$ is defined such that there are two radii $r_a$ and $r_b$ for which a radial function of change in optical refraction power $B_{COR}(r,\varphi)$ is piecewise constant, for which thus $B_{COR}(r<r_a,\varphi=const) = B_a \neq B_b = B_{COR}(r>r_b,\varphi=const)$ holds true, wherein the radial function of the change in optical refraction power $B_{COR}(r,\varphi)$ changes continuously from $B_a$ to $B_b$ in a transition area between $r_a$ and $r_b$.

7. The method according to any of claims 3-6, **characterized in that**, when generating the control data, the local change in optical refraction power is defined to be angle-independent.

8. An apparatus for generating control data which are adapted to control a laser device (L) for surgical correction of defective vision of an eye (3) of a patient (4), wherein during operation of the laser device (L) the cornea (5) is deformed by pressing its front surface (15) against a contact surface and wherein a correction surface is predetermined for the non-deformed cornea (5), which correction surface is to be produced as a cut surface in the cornea for correcting defective vision, and

- wherein the apparatus, when generating the control data, is adapted to consider the deformation of the cornea (5) during operation of the laser device (L) by a transformation (AT) of the coordinates of points in the non-deformed cornea (5) into coordinates of the same points in the deformed cornea (5),

**characterized in that**

a) the apparatus, when generating the control data, is adapted to select a subset of points ($f_i$) lying in the correction surface (F) or in an approximation surface (N) derived therefrom and to transform the coordinates of the subset of selected points by means of the transformation in order to obtain transformed points ($f_i'$),
b) the apparatus, when generating the control data, is configured to fit an interpolation surface (I') to the transformed points ($f_i'$) by interpolation and
c) the apparatus, when generating the control data, is adapted to select target points lying on the interpolation surface (I') and to use them for generating the control data.

9. The apparatus according to claim 8, **characterized in that** the apparatus, when generating the control data, is adapted to derive the approximation surface (N) from the correction surface (F) by a smoothing or approximation process prior to step a), and to determine a deviation (D) between the correction surface (F) and the approximation surface (N) and to correct the interpolation surface (I') by using this deviation (D) prior to step c).

10. The apparatus according to claim 8, **characterized in that**

- the control data are adapted to control a laser device (L) which severs corneal tissue by irradiating laser radiation (2) into the cornea (5) of the eye (3),
- the apparatus (12) is adapted to generate the control data such that under operation according to the control data the laser device (L) emits the laser radiation (2) such that a volume (18) is isolated in the cornea (5), the removal of which volume from the cornea (5) effects the desired correction of the defective vision and,
- to determine the correction surface, the apparatus (12) is adapted to calculate

- - either a radius of curvature $R_{CV}^*$ which radius the cornea (5) has without the volume (18), and which is locally varying and satisfies the equation

$$R_{CV}^*(r,\varphi) = 1 / ( (1/R_{CV}(r,\varphi)) + B_{COR}(r,\varphi)/ (n_c-1) ) + F,$$

wherein $R_{CV}(r,\varphi)$ is the local radius of curvature of the cornea (5) before the volume (18) is removed, $n_c$ is the refractive index of the material of the cornea (5), F is a coefficient, and $B_{COR}(r,\varphi)$ is the local change in optical refraction power in a plane lying in the vertex of the cornea (5) and required for the desired correction of the defective vision,
- - or an optical refraction power $B_{CV}^*$ which the cornea (5) has without the volume (18), and which is locally varying and satisfies the equation

$$B^*_{CV}(r,\varphi) = \cfrac{1}{\cfrac{1}{B_{CV}(r,\varphi) + B_{COR}(r,\varphi)} + \cfrac{F}{(n_C - 1)}}$$

wherein $B_{CV}(r,\varphi)$ is the local optical refraction power of the cornea (5) before the volume (18) is removed, $n_c$ is the refractive index of the material of the cornea (5), F is a coefficient, and $B_{COR}(r,\varphi)$ is the local change in optical refraction power defined in a plane lying in the vertex of the cornea (5) and required for the desired correction of the defective vision,

- wherein there are at least two radii, r1 and r2, for which $B_{COR}(r=r1,\varphi) \neq B_{COR}(r=r2,\varphi)$ holds true.

11. The apparatus according to claim 10, **characterized in that** the apparatus (12) is adapted to use $F = (1 - 1/n_c) \cdot \Delta(r=0,\varphi)$ for the coefficient F when generating the control data, wherein $\Delta z(r=0,\varphi)$ is the central thickness of the volume (18) to be isolated in the cornea (5) and to be removed, and the apparatus is adapted to iteratively calculate the local radius of curvature $R_{CV}^*(r,\varphi)$, wherein the apparatus derives in each iteration step the central thickness $\cdot \Delta z(r=0,\varphi)$ of the volume (18) from the difference between the central reciprocal radii of curvature $1/R_{CV}^*(r=0,\varphi)$ and $1/R_{CV}(r=0,\varphi)$, and uses this value when calculating $R_{CV}^*(r,\varphi)$ in the next iteration step.

12. The apparatus according to claim 10 or 11, **characterized in that**, when generating the control data, the apparatus defines the local change in optical refraction power $B_{COR}(r,\varphi)$ such that there is a characteristic radius $r_{ch}$ for which a radial function of the change in optical refraction power $B_{COR}(r,\varphi)$ is piecewise constant, for which radii thus $B_{COR}(r<r_{ch},\varphi=const) = B_a \neq B_b = B_{COR}(r>r_{ch},\varphi=const)$ holds true.

13. The apparatus according to claim 10 or 11, **characterized in that**, when generating the control data, the apparatus defines the local change in optical refraction power $B_{COR}(r,\varphi)$ such that there are two radii $r_a$ and $r_b$ for which the radial function of change in optical refraction power $B_{COR}(r,\varphi)$ is piecewise constant, for which radii thus $B_{COR}(r<r_a,\varphi=const) = B_a \neq B_b = B_{COR}(r>r_b,\varphi=const)$ holds true, wherein the radial function of the change in optical refraction power $B_{COR}(r,\varphi)$ changes continuously from $B_a$ to $B_b$ in a transition area between $r_a$ and $r_b$.

14. The apparatus according to any of claims 10-13, **characterized in that**, when generating the control data, the apparatus defines the local change in optical refraction power to be angle-independent.

15. A computer program product with program code which carries out a method according to any of claims 1-7 when executed on a computer.

**Revendications**

1. Procédé pour générer des données de commande, lesquelles sont conçues pour commander un appareil à laser (L) en vue de la correction d'amétropie opérative d'un oeil (3) d'un patient (4), la cornée (5) étant déformée lors du fonctionnement de l'appareil à laser (L) par pressage de sa surface avant (15) contre une surface de contact, une surface de correction rapportée à la cornée (5) non déformée étant prédéfinie dans le procédé, laquelle est générée en tant que surface de coupe dans la cornée en vue de la correction d'amétropie, et

- la déformation de la cornée (5) présente lors du fonctionnement de l'appareil à laser (L) étant prise en compte dans le procédé par une transformation (AT) des coordonnées de points dans la cornée (5) non déformée en coordonnées des mêmes points dans la cornée (5) déformée,

**caractérisé en ce que**

a) une quantité partielle des points ($f_i$) qui se trouvent dans la surface de correction (F) ou dans une surface d'approximation (N) qui en est dérivée est sélectionnée et leurs coordonnées sont transformées au moyen de la transformation afin d'obtenir des points transformés (fi'),

b) une surface d'interpolation (I') est adaptée par interpolation aux points transformés (fi') et

c) les points cibles se trouvant sur la surface d'interpolation (I') sont sélectionnés et utilisés pour générer les données de commande.

2. Procédé selon la revendication 1, **caractérisé en ce qu'**avant l'étape a), la surface d'approximation (N) est dérivée à partir de la surface de correction (F) par une opération de lissage ou d'approximation, et **en ce qu'**avant l'étape c), un écart (D) entre la surface de correction (F) et la surface d'approximation (N) est déterminé et la surface d'interpolation (I') est corrigée à l'aide de cet écart (D).

3. Procédé selon la revendication 1 ou 2, **caractérisé en ce que**

- les données de commande sont configurées pour commander un appareil à laser (L) qui sépare le tissu cornéen par injection du rayonnement laser (2) dans la cornée (5) de l'oeil (3),
- les données de commande sont créées de telle sorte que l'appareil à laser (L), lors du fonctionnement, délivre le rayonnement laser (2) conformément aux données de commande de telle sorte qu'un volume (18) est isolé dans la cornée (5), dont l'enlèvement de la cornée (5) produit la correction d'amétropie souhaitée, et
- l'un des éléments suivants est calculé en vue de déterminer la surface de correction :

-- soit un rayon de courbure $R_{CV}{}^*$ que possède la cornée (5) réduite du volume (18), lequel est dépendant de l'endroit et vérifie l'équation suivante :

$$R_{CV}{}^*(r, \varphi) = 1/((1/R_{CV}(r, \varphi)) + B_{COR}(r, \varphi)/(n_c-1)) + F,$$

où $R_{CV}(r, (\varphi))$ désigne le rayon de courbure local de la cornée (5) avant l'enlèvement du volume (18), $n_c$ la réfringence du matériau de la cornée (5), F un facteur et où $B_{COR}(r, (\varphi))$ désigne la modification de la réfringence locale nécessaire pour la correction d'amétropie souhaitée dans un plan qui se trouve dans le sommet de la cornée (5),

-- soit une réfringence $B_{CV}{}^*$ que possède la cornée (5) réduite du volume (18) et qui vérifie l'équation suivante :

$$B^*{}_{CV}(r, \varphi) = \frac{1}{\frac{1}{B_{CV}(r,\varphi) + B_{COR}(r,\varphi)} + \frac{F}{(n_c-1)}}$$

où $B_{CV}(r, (\varphi))$ désigne la réfringence locale de la cornée (5) avant l'enlèvement du volume (18), $n_c$ la réfringence du matériau de la cornée (5), F un facteur et où $B_{COR}(r, (\varphi))$ désigne la modification de la réfringence locale nécessaire pour la correction d'amétropie souhaitée dans un plan qui se trouve dans le sommet de la cornée (5),

- au moins deux rayons r1 et r2 étant présents, pour lesquels la relation $B_{COR}(r=r1, \varphi) \neq B_{COR}(r=r2, \varphi)$ est vérifiée.

4. Procédé selon la revendication 3, **caractérisé en ce que** lors de la détermination des données de commande, F = $(1 - 1/n_c) * \Delta z(r=0, \varphi)$ est utilisé pour le facteur, $\Delta z(r=0, \varphi)$ désignant l'épaisseur centrale du volume (18) à isoler et à enlever dans la cornée (5), et le rayon de courbure dépendant de l'endroit $R_{CV}{}^*(r, \varphi)$ est calculé de manière itérative **en ce que** lors de chaque étape de l'itération, l'épaisseur centrale $\Delta z(r=0, \varphi)$ du volume (18) est déduite à partir de la différence entre les rayons de courbure centraux réciproques $1/R_{CV}{}^*(r=0, \varphi)$ et $1/R_{CV}(r=0, \varphi)$ et cette valeur est utilisée lors du calcul de $R_{CV}{}^*(r, \varphi)$ dans la prochaine étape de l'itération.

5. Procédé selon la revendication 3 ou 4, **caractérisé en ce que** lors de la détermination des données de commande, la modification de la réfringence locale $B_{COR}(r, \varphi)$ est fixée de telle sorte qu'il existe un rayon caractéristique $r_{ch}$ pour lequel la fonction radiale de la modification de la réfringence $B_{COR}(r, \varphi)$ est constante par segments et pour lequel la relation $B_{COR}(r<r_{ch}, \varphi=const) = B_a \neq \neq B_b = B_{COR}(r>r_{ch}, \varphi=const)$ est ainsi vérifiée.

6. Procédé selon la revendication 3 ou 4, **caractérisé en ce que** lors de la détermination des données de commande, la modification de la réfringence locale $B_{COR}(r, \varphi)$ est fixée de telle sorte qu'il existe un deux rayons $r_a$ et $r_b$ pour lesquels la fonction radiale de la modification de la réfringence $B_{COR}(r, \varphi)$ est constante par segments et pour lequels la relation $B_{COR}(r<r_a, \varphi=const) = B_a \neq B_b = B_{COR}(r>r_b, \varphi=const)$ est ainsi vérifiée, la fonction radiale de la modification de la réfringence $B_{COR}(r, \varphi)$ dans la zone de transition entre $r_a$ et $r_b$ passant continuellement de $B_a$ à $B_b$.

**7.** Procédé selon l'une des revendications 3 à 6, **caractérisé en ce que** lors de la détermination des données de commande, la modification de la réfringence locale est fixée indépendamment de l'angle.

**8.** Dispositif pour générer des données de commande, lesquelles sont conçues pour commander un appareil à laser (L) en vue de la correction d'amétropie opérative d'un oeil (3) d'un patient (4), la cornée (5) étant déformée lors du fonctionnement de l'appareil à laser (L) par pressage de sa surface avant (15) contre une surface de contact et une surface de correction rapportée à la cornée (5) non déformée étant prédéfinie, laquelle est à générer en tant que surface de coupe dans la cornée en vue de la correction d'amétropie, et

- le dispositif étant conçu pour, lors de la génération des données de commande, prendre en compte la déformation de la cornée (5) présente lors du fonctionnement de l'appareil à laser (L) par une transformation (AT) des coordonnées de points dans la cornée (5) non déformée en coordonnées des mêmes points dans la cornée (5) déformée,

**caractérisé en ce que**

a) le dispositif est configuré pour, lors de la génération des données de commande, sélectionner une quantité partielle des points (fi) qui se trouvent dans la surface de correction (F) ou dans une surface d'approximation (N) qui en est dérivée et transformer leurs coordonnées au moyen de la transformation afin d'obtenir des points transformés (fi'),

b) le dispositif est configuré pour, lors de la génération des données de commande, adapter une surface d'interpolation (I') par interpolation aux points transformés (fi') et

c) le dispositif est configuré pour, lors de la génération des données de commande, sélectionner les points cibles se trouvant sur la surface d'interpolation (I') et les utiliser pour générer les données de commande.

**9.** Dispositif selon la revendication 8, **caractérisé en ce que** le dispositif est configuré pour, lors de la génération des données de commande, avant l'étape a), dériver la surface d'approximation (N) à partir de la surface de correction (F) par une opération de lissage ou d'approximation et, avant l'étape c), déterminer un écart (D) entre la surface de correction (F) et la surface d'approximation (N) et corriger la surface d'interpolation (I') à l'aide de cet écart (D).

**10.** Dispositif selon la revendication 8, **caractérisé en ce que**

- les données de commande sont configurées pour commander un appareil à laser (L) qui sépare le tissu cornéen par injection du rayonnement laser (2) dans la cornée (5) de l'oeil (3),

- le dispositif (12) est conçu pour créer les données de commande de telle sorte que l'appareil à laser (L), lors du fonctionnement, délivre le rayonnement laser (2) conformément aux données de commande de telle sorte qu'un volume (18) est isolé dans la cornée (5), dont l'enlèvement de la cornée (5) produit la correction d'amétropie souhaitée, et

- le dispositif (12) est conçu pour calculer l'un des éléments suivants en vue de déterminer la surface de correction :

-- soit un rayon de courbure $R_{CV}^*$ que possède la cornée (5) réduite du volume (18), qui est dépendant de l'endroit et qui vérifie l'équation suivante :

$$R_{CV}^*(r, \varphi)=1/((1/R_{CV}(r, \varphi)) + B_{COR}(r, \varphi)/(n_c-1)) + F,$$

où $R_{CV}(r, \varphi)$ désigne le rayon de courbure local de la cornée (5) avant l'enlèvement du volume (18), $n_c$ la réfringence du matériau de la cornée (5), F un facteur et $B_{COR}(r, \varphi)$ désigne la modification de la réfringence locale nécessaire pour la correction d'amétropie souhaitée dans un plan qui se trouve dans le sommet de la cornée (5),

-- soit une réfringence $B_{CV}^*$ que possède la cornée (5) réduite du volume (18) et qui vérifie l'équation suivante :

$$B^*{}_{CV}(r, \varphi) = \cfrac{1}{\cfrac{1}{B_{CV}(r,\varphi) + B_{COR}(r,\varphi)} + \cfrac{F}{(n_c - 1)}}$$

où $B_{CV}(r, \varphi)$ désigne la réfringence locale de la cornée (5) avant l'enlèvement du volume (18), $n_c$ la réfringence du matériau de la cornée (5), F un facteur et $B_{COR}(r, \varphi)$ désigne la modification de la réfringence locale nécessaire pour la correction d'amétropie souhaitée dans un plan qui se trouve dans le sommet de la cornée (5),

- au moins deux rayons r1 et r2 étant présents, pour lesquels la relation $B_{COR}(r{=}r1, \varphi) \neq B_{COR}(r{=}r2, \varphi)$ est vérifiée.

11. Dispositif selon la revendication 10, **caractérisé en ce que** le dispositif (12) est configuré pour, lors de la détermination des données de commande, utiliser $F = (1 - 1/n_c) * \Delta z(r{=}0, \varphi)$ pour le facteur, $\Delta z(r{=}0, \varphi)$ désignant l'épaisseur centrale du volume (18) à isoler et à enlever dans la cornée (5), et le dispositif est configuré pour calculer de manière itérative le rayon de courbure dépendant de l'endroit $R_{CV}^*(r, \varphi)$ **en ce que** lors de chaque étape de l'itération, il déduit l'épaisseur centrale $\Delta z(r{=}0, \varphi)$ du volume (18) à partir de la différence entre les rayons de courbure centraux réciproques $1/R_{CV}^*(r{=}0, \varphi)$ et $1/R_{CV}(r{=}0, \varphi)$ et utilise cette valeur lors du calcul de $R_{CV}^*(r, \varphi)$ dans la prochaine étape de l'itération.

12. Dispositif selon la revendication 10 ou 11, **caractérisé en ce que** lors de la détermination des données de commande, il fixe la modification de la réfringence locale $B_{COR}(r, \varphi)$ de telle sorte qu'il existe un rayon caractéristique $r_{ch}$ pour lequel la fonction radiale de la modification de la réfringence $B_{COR}(r, \varphi)$ est constante par segments et pour lequel la relation $B_{COR}(r{<}r_{ch}, \varphi{=}const) = B_a \neq B_b = B_{COR}(r{>}r_{ch}, \varphi{=}const)$ est ainsi vérifiée.

13. Dispositif selon la revendication 10 ou 11, **caractérisé en ce que** lors de la détermination des données de commande, il fixe la modification de la réfringence locale $B_{COR}(r, \varphi)$ de telle sorte qu'il existe un deux rayons $r_a$ et $r_b$ pour lesquels la fonction radiale de la modification de la réfringence $B_{COR}(r, \varphi)$ est constante par segments et pour lequel la relation $B_{COR}(r{<}r_a, \varphi{=}const) = B_a \neq B_b = B_{COR}(r{>}r_b, \varphi{=}const)$ est ainsi vérifiée, la fonction radiale de la modification de la réfringence $B_{COR}(r, \varphi)$ dans la zone de transition entre $r_a$ et rb passant continuellement de $B_a$ à $B_b$.

14. Dispositif selon l'une des revendications 10 à 13, **caractérisé en ce que** lors de la détermination des données de commande, il fixe la modification de la réfringence locale indépendamment de l'angle.

15. Produit de programme informatique comprenant un code de programme qui, lors de l'exécution sur un ordinateur, met en oeuvre un procédé selon l'une des revendications 1 à 7.

Fig. 1

Fig. 2

Fig. 3

Fig. 4

Fig. 5

Fig. 6

Fig. 7

Fig. 8

Fig. 9

EP 2 389 147 B1

Fig. 10

Diagnose Daten →

**S1**
Topographie
der Kornea

$Z_{CV}(r,\varphi)$

Diagnose Daten →
(optional)

**S2**
Lokale
Krümmung

$R_{CV}(r,\varphi)$

**S3**
Lokale
Brechkraft

$B_{CV}(r,\varphi)$

Daten zur refraktiven →
Korrektur

**S4**
Lokale Brechkraft nach
Korrektur

$B^*_{CV}(r,\varphi)=B_{CV}(r,\varphi)+B_{COR}(r,\varphi)$

**S5**
Neuer lokaler
Krümmungsradius

$R_{CV}^*(r,\varphi)$

**S6**
Volumenbegrenzung
muss Krümmungsänderung umsetzen

↙ Vorgabe der Grundform
des Volumens

**S7**
Veränderung der
Topographie der Kornea

$Z_{CV}^*(r,\varphi)=Z_{CV}(r,\varphi)+\Delta Z(r,\varphi)$

Fig. 11

Fig. 12

**IN DER BESCHREIBUNG AUFGEFÜHRTE DOKUMENTE**

**In der Beschreibung aufgeführte Patentdokumente**

- WO 9611655 A
- EP 1153584 A1
- US 5993438 A
- WO 2005092172 A
- US 6110166 A
- US 7131968 B
- DE 102006053118 A1
- DE 102006053120 A1
- DE 102006053119 A1
- WO 2005011547 A1

- US 20080319428 A1
- WO 2008055706 A
- DE 102008017293 A1
- EP 1159986 A1
- US 5549632 A
- DE 69500997 T2
- WO 2004032810 A2
- US 20070293851 A1
- US 2008234707 A1
- US 20070293851 A